# EUROPEAN PATENT APPLICATION

(11) **EP 4 375 366 A1**
(43) Date of publication of application: **29.05.2024**
(21) Application number: 22845457.5
(22) Date of filing: 22.07.2022
(51) Int. Cl.: C12N 1/21, C12N 15/52, C12N 15/31, C12P 19/02

(54) **METHOD FOR PRODUCING GLUCOSE AND DERIVATIVES THEREOF BY MEANS OF BIOTRANSFORMATION WITH RECOMBINANT YEAST**

(30) Priority: 22.07.2021 WO PCT/CN2021/107787
(71) Applicant: Shenzhen Institutes of Advanced Technology, Shenzhen, Guangdong 518055 (CN)
(72) Inventor: YU, Tao, Shenzhen, Guangdong 518055 (CN); WU, Lianghuan, Shenzhen, Guangdong 518055 (CN); TANG, Hongting, Shenzhen, Guangdong 518055 (CN); GUO, Shuyuan, Shenzhen, Guangdong 518055 (CN)
(74) Representative: Grünecker Patent- und Rechtsanwälte PartG mbB
(86) International application number: PCT/CN2022/107431
(87) International publication number: WO 2023/001294

(57) **Abstract**

A method for producing glucose and derivatives thereof by means of biotransformation with a recombinant yeast, which belongs to the technical field of synthetic biology. A construction method comprises any one of the following steps: i, knocking out metabolic pathway-related enzymes of glucose and derivatives thereof in a yeast strain; ii, enhancing or using an activity of synthetic pathway-related enzymes of glucose and derivatives thereof in the yeast strain; and iii, enhancing or using a capability of glucose and derivatives thereof in the yeast strain to enter and exit the yeast. Further provided are a recombinant yeast strain capable of producing glucose or derivatives thereof at a high yield and the use thereof in the conversion of a non-grain low-carbon carbon source. The low-carbon non-grain carbon source synthesized by means of using photoelectrocatalysis or traditional chemical industry is used as a substrate, and rapid preparation of food product raw materials glucose and derivatives thereof from the non-grain carbon source is realized by means of recombinant yeast cells.

## Description

### TECHNICAL FIELD

The present disclosure belongs to the technical field of synthetic biology and specifically relates to a method for producing glucose and derivatives thereof by means of biotransformation with a recombinant yeast.

### BACKGROUND

Glucose, with the chemical formula C₆H₁₂O₆, is the most widely distributed and most important hexose in nature. As an important food and chemical raw material, glucose has been widely used in the fields of food, fermentation, and medicine. At present, glucose is mainly prepared by using starch extracted from corn, potatoes, and other food crops as raw materials, and the main production processes include: an acid method, an acid enzyme method, and a double-enzymatic method, among which the double-enzymatic method is currently the most ideal and widely used. Starch is hydrolyzed into form glucose after steps such as gelatinization, liquefaction, and saccharification, and such a production process is complicated, requires large energy consumption, and consumes a large amount of food crops. China is a country with a large population, and by 2020, China's population has reached 1.4 billion, accounting for 20% of the world's population. However, the total arable land area accounts for only 7% of the world's total, the arable land per capita is less than 40% of the world's arable land per capita, and the arable land productivity is near the limit. In addition, the grain production process requires a long period of time and is easily affected by regional, climatic, and war factors, and the food crisis is becoming more severe. Therefore, a new method for producing glucose needs to be developed urgently to effectively avoid the problems described above and implement large-scale and rapid preparation of the food raw material glucose.

In addition to glucose, the present disclosure further extends the chemical space of sugar derivatives to produce monosaccharide derivatives (inositol), glucosamine, and polysaccharide derivatives (sucrose).

Inositol is essential to health and its supplements have been proven to improve metabolic performance as a valuable alternative to the treatment of multiple diseases. At present, the production methods of inositol mainly include a hydrolysis method and a chemical synthesis method, but these methods have problems such as a low yield, high energy consumption, and serious pollution.

Glucosamine has a variety of specific biological activities and has been widely used in industries of food, cosmetics, and biomedicines. The production methods of glucosamine mainly include an acid hydrolysis method, an enzymolysis method, and a microbiological fermentation method, and the first two methods have been gradually eliminated due to factors such as low production efficiency and environmental pollution.

Oligosaccharides and polysaccharides play a major role in nutrition and are the structural components of living organisms. Sucrose is a well-known oligosaccharide and has been widely used in industries such as food, medicines and bulk chemical industries. At present, sucrose is mainly extracted from sugarcane in tropical regions and sugar beets in temperate regions. Polysaccharide starch is the main source of food energy and it has a wide range of applications in the synthesis of paper and biodegradable materials. In the past, farming was the only way to produce starch. Recently, starch synthesis *in vitro* has been achieved using CO₂ as a carbon source in a cell-free system based on a chemical-biochemical approach. Therefore, with a low-carbon compound obtained by electrochemically transforming CO₂ as a carbon source, glucose and sugar derivatives are produced through microbial cell factories, thereby effectively using CO₂ and providing a new strategy for grain supply.

### SUMMARY

An object of the present disclosure is to provide a method for constructing a recombinant yeast strain.

Another object of the present disclosure is to provide a recombinant yeast strain for producing glucose and derivatives thereof and strains.

Another object of the present disclosure is to provide a recombinant *Saccharomyces cerevisiae* strain for producing glucose and derivatives thereof in a high yield.

Another object of the present disclosure is to provide the use of a strain for producing glucose and derivatives thereof in a high yield in the transformation of non-grain low-carbon carbon sources.

In a first aspect, the present disclosure provides a method for constructing a recombinant yeast strain for the production of glucose and derivatives thereof, which includes any one of the following construction methods:
i, knocking out metabolic pathway-related enzymes of glucose and derivatives thereof in a yeast strain;
ii, enhancing or using the activity of synthetic pathway-related enzymes of glucose and derivatives thereof in the yeast strain; and
iii, enhancing or using the capability of glucose and derivatives thereof in the yeast strain to enter and exit the yeast.

Preferably, the yeast strain includes *Saccharomyces cerevisiae, Pichia pastoris,* and a *Yarrowia lipolytica,* more preferably, *Saccharomyces cerevisiae* and *Pichiapastoris.*

In the construction method, the metabolic pathway-related enzymes of glucose include glucokinase and related hexokinase isoenzymes; and the metabolic pathway-related enzymes of sucrose in the glucose derivatives include sucrase, maltase, and isomaltase.

The synthetic pathway-related enzymes of glucose include glucose phosphatase, and preferably, glucose phosphatase includes glucose-1-phosphatase and glucose-6-phosphatase; the synthetic pathway-related enzymes of glucosamine in the glucose derivatives include glucosamine-6-phosphate phosphatase and glucosamine-6-phosphate deaminase; the synthetic pathway-related enzymes of sucrose in the glucose derivatives include sucrose phosphate phosphatase, sucrose phosphate synthase, UDP-glucose pyrophosphorylase, and ADP-glucose pyrophosphorylase; the synthetic pathway-related enzymes of inositol in the glucose derivatives include inositol-3-phosphate synthase and inositol monophosphatase.

A protein that enhances the capability of sucrose in the glucose derivatives to enter and exit the yeast includes a sucrose transporter, and a protein that enhances the capability of inositol in the glucose derivatives to enter and exit the yeast includes an inositol transporter.
1) In a preferred embodiment, a method for constructing a recombinant *Saccharomyces cerevisiae* strain for producing glucose includes, but is not limited to: A, abolishing the activity of metabolic pathway-related enzymes of glucose in the yeast strain; B, using the activity of synthetic pathway-related enzymes of glucose in the yeast itself; and C, using the capability of glucose in the yeast itself to enter and exit the yeast.
2) In a preferred embodiment, a method for constructing a recombinant *Pichiapastoris* strain for producing glucose includes, but is not limited to: A, abolishing the activity of metabolic pathway-related enzymes of glucose in the yeast strain; B, using the activity of synthetic pathway-related enzymes of glucose in the yeast itself; and C, using the capability of glucose in the yeast itself to enter and exit the yeast.
3) In a preferred embodiment, a method for constructing a recombinant yeast strain for producing glucosamine includes, but is not limited to: A, abolishing the activity of metabolic pathway-related enzymes of glucosamine in the yeast strain; B, enhancing the activity of synthetic pathway-related enzymes of glucosamine in the yeast; and C, using the capability of glucosamine in the yeast itself to enter and exit the yeast.
4) In a preferred embodiment, a method for constructing a recombinant yeast strain for producing sucrose includes, but is not limited to: A, abolishing the activity of metabolic pathway-related enzymes of sucrose in the yeast strain; B, enhancing the activity of synthetic pathway-related enzymes of sucrose; and C, using the capability of glucose and derivatives thereof in the yeast itself to enter and exit the yeast.
5) In a preferred embodiment, a method for constructing a recombinant yeast strain for producing inositol includes, but is not limited to: A, abolishing the activity of metabolic pathway-related enzymes of glucose in the yeast strain; B, enhancing the activity of synthetic pathway-related enzymes of inositol; and C, using the capability of inositol in the yeast itself to enter and exit the yeast.

In a second aspect, the present disclosure provides a recombinant *Saccharomyces cerevisiae* strain for producing glucose and derivatives thereof in a high yield.

The recombinant *Saccharomyces cerevisiae* strain includes any one of the following recombinant yeast strains:
1) a recombinant yeast strain A with a glucose utilization deficiency and a capability to secrete glucose, which is obtained by knocking out encoding genes of glucokinase and related hexokinase isoenzymes in *Saccharomyces cerevisiae*/*Pichia pastoris*;
2) a recombinant yeast strain B with a capability to secrete glucosamine, which is obtained by inserting several copies of glucosamine-6-phosphate phosphatase GlmP and glucosamine-6-phosphate deaminase GlmD into a recombinant yeast strain E with an improved glucose synthesis yield which is used as a starting strain, where the recombinant yeast strain E is obtained by optionally knocking out hexokinase isoenzyme genes in the recombinant yeast strain A and overexpressing glucose phosphatase and HAD4 of *Escherichia coli* or overexpressing HAD4 of *Escherichia coli,* and preferably, three copies of GlmD and three copies of GlmP are inserted or three copies of GlmD and four copies of GlmP are inserted;
3) a recombinant yeast strain C with a sucrose utilization deficiency and a capability to secrete sucrose, which is obtained by knocking out encoding genes of sucrase, maltase, and isomaltase in a yeast, inserting a sucrose transporter SUF 1, and inserting one or more copies of sucrose phosphate phosphatase SPP and sucrose phosphate synthase SPS;
4) a recombinant yeast strain D with an inositol utilization deficiency and a capability to secrete inositol, which is obtained by inserting endogenous inositol-3-phosphate synthase INO1 and exogenous inositol monophosphatase SuhB into a yeast and inserting an inositol transporter ITR1;
wherein, inositol-3-phosphate synthase INO1 is derived from *Saccharomyces cerevisiae* itself; inositol monophosphatase SuhB is derived from *Escherichia coli*; the inositol transporter ITR1 and glutamate transhydrogenase GDH1 are derived from *Saccharomyces cerevisiae.*

Preferably, the yeast includes *Saccharomyces cerevisiae, Pichia pastoris,* and a *Yarrowia lipolytica.*

For the recombinant yeast strain, a specific method for constructing the recombinant yeast strain A described in 1) is: when *Saccharomyces cerevisiae* is selected, knocking out a glucokinase gene *glkl* having a glucokinase activity and two hexokinase isoenzyme genes *hxk1* and *hxk2* in *Saccharomyces cerevisiae* to obtain a recombinant *Saccharomyces cerevisiae* strain A with a glucose utilization deficiency and a capability to secrete glucose; and
when *Pichia pastoris* is selected, knocking out a glucokinase gene *glkl* having a glucokinase activity and a hexokinase isoenzyme gene *hxkl* in *Pichiapastoris* to obtain a recombinant *Pichia pastoris* strain A with a glucose utilization deficiency and a capability to secrete glucose.

A specific method for constructing the recombinant yeast strain C described in 3) is: when the yeast is *Saccharomyces cerevisiae,* knocking out a sucrase active gene *suc2,* maltase active genes *mal12, mal22,* and *mal32,* and isomaltase active genes *ims1, ima2, ima3, ima4,* and *ima5* to obtain a recombinant *Saccharomyces cerevisiae* strain with a sucrose utilization deficiency, and overexpressing a sucrose transporter SUF1 from pea and sucrose phosphate phosphatase SPP and sucrose phosphate synthase SPS from polycystis to obtain a recombinant yeast strain C with a sucrose utilization deficiency and a capability to secrete sucrose.

The recombinant yeast strain preferably is:
a) the recombinant yeast strain E with an improved glucose synthesis yield, which is obtained by optionally knocking out hexokinase isoenzyme genes in the recombinant yeast strain A and overexpressing glucose phosphatase and HAD4 of *Escherichia coli* or overexpressing HAD4 of *Escherichia coli;*
b) a recombinant yeast strain F with an improved glucosamine synthesis yield, which is obtained by knocking out a yeast endogenous gene *reg1* in the recombinant yeast strain B;
c) a recombinant yeast strain G with an improved sucrose synthesis yield, which is obtained by inserting a glucose pyrophosphorylase GlgC mutant and UGP1 into the recombinant yeast strain C and increasing precursor substances ADP-Glc and UDP-Glc, respectively;
d) a recombinant yeast strain H with an improved inositol synthesis yield, which is obtained by knocking out phosphofructokinase 1 and phosphofructokinase 2 in the recombinant yeast strain D and overexpressing glutamate transhydrogenase GDH1.

For the recombinant yeast strain, glucose phosphatase possesses glucose-1-phosphate and/or glucose-6-phosphate activities, and glucose phosphatase, glucose pyrophosphorylase GlgC/UGP1, glutamate transhydrogenase, glucosamine-6-phosphate phosphatase GlmP, glucosamine-6-phosphate deaminase GlmD, sucrose phosphate phosphatase SPP, sucrose phosphate synthase SPS, sucrose transporter, inositol-3-phosphate synthase, inositol monophosphatase, and inositol transporter are derived from heterologous enzymes or specific modified enzymes of the yeast itself or other eukaryotic and prokaryotic organisms.

For the recombinant yeast strain, a specific method for constructing the recombinant yeast strain E described in a) is: knocking out hexokinase genes *emi2* and *YLR446W* in a recombinant *Saccharomyces cerevisiae* A with a glucose utilization deficiency and a capability to secrete glucose and overexpressing a glucose phosphatase gene *agpP* from *Pantoea* and an HAD4 gene *yihx* from *Escherichia coli* at *YLR446W* and *emi2* sites, respectively, or knocking out hexokinase genes *emi2* and *YLR446W* in a recombinant *Saccharomyces cerevisiae* A with a glucose utilization deficiency and a capability to secrete glucose and overexpressing an HAD4 gene *yihx* from *Escherichia coli* at an *emi2* site, to obtain the recombinant yeast strain E with an improved glucose synthesis yield.

A specific method for constructing the recombinant yeast strain E described in a) is: when the recombinant yeast strain A is constructed by selecting *Pichia pastoris* as an original yeast, knocking out hexokinase isoenzyme genes *hxk2* and *hxk iso2* and overexpressing an HAD4 gene *yihx* from *Escherichia coli* at an *hxk iso2* site to obtain the recombinant yeast strain E with an improved glucose synthesis yield.

A specific method for constructing the recombinant yeast strain G described in c) is: overexpressing UDP-glucose pyrophosphorylase UGP1 derived from *Saccharomyces cerevisiae* and a mutant of ADP-glucose pyrophosphorylase GlgC derived from *Escherichia coli* to obtain the recombinant *Saccharomyces cerevisiae* strain G for producing sucrose thereof in a high yield.

For the recombinant yeast strain, the nucleotide sequence of *agpP* is as shown in SEQ ID NO. 1, or a sequence having at least 70% homology therewith; the amino acid sequence of a protein formed after the expression of *agpP* is shown in SEQ ID NO. 32, or a sequence having at least 70% homology therewith.

The nucleotide sequence of *yihx* is as shown in SEQ ID NO. 2, or a sequence having at least 70% homology therewith; the amino acid sequence of a protein formed after the expression of *yihx* is shown in SEQ ID NO. 33, or a sequence having at least 70% homology therewith.

The nucleotide sequence of GlmD is as shown in SEQ ID NO. 17, or a sequence having at least 70% homology therewith; the amino acid sequence of a protein formed after the expression of GlmD is shown in SEQ ID NO. 34, or a sequence having at least 70% homology therewith.

The nucleotide sequence of GlmP is as shown in SEQ ID NO. 18, or a sequence having at least 70% homology therewith; the amino acid sequence of a protein formed after the expression of GlmP is shown in SEQ ID NO. 35, or a sequence having at least 70% homology therewith.

The nucleotide sequence of SUF 1 is as shown in SEQ ID NO. 7, or a sequence having at least 70% homology therewith; the amino acid sequence of a protein formed after the expression of SUF 1 is shown in SEQ ID NO. 36, or a sequence having at least 70% homology therewith.

The nucleotide sequence of SPP is as shown in SEQ ID NO. 9, or a sequence having at least 70% homology therewith; the amino acid sequence of a protein formed after the expression of SPP is shown in SEQ ID NO. 37, or a sequence having at least 70% homology therewith.

The nucleotide sequence of SPS is as shown in SEQ ID NO. 10, or a sequence having at least 70% homology therewith; the amino acid sequence of a protein formed after the expression of SPS is shown in SEQ ID NO. 38, or a sequence having at least 70% homology therewith.

The nucleotide sequence of UGP1 is as shown in SEQ ID NO. 12, or a sequence having at least 70% homology therewith; the amino acid sequence of a protein formed after the expression of UGP1 is shown in SEQ ID NO. 39, or a sequence having at least 70% homology therewith.

The nucleotide sequence of the GlgC mutant is as shown in SEQ ID NO. 13, or a sequence having at least 70% homology therewith; the amino acid sequence of a protein formed after the expression of the GlgC mutant is shown in SEQ ID NO. 40, or a sequence having at least 70% homology therewith.

The nucleotide sequence of INO1 is as shown in SEQ ID NO. 26, or a sequence having at least 70% homology therewith; the amino acid sequence of a protein formed after the expression of INO1 is shown in SEQ ID NO. 41, or a sequence having at least 70% homology therewith.

The nucleotide sequence of ITR1 is as shown in SEQ ID NO. 27, or a sequence having at least 70% homology therewith; the amino acid sequence of a protein formed after the expression of ITR1 is shown in SEQ ID NO. 42, or a sequence having at least 70% homology therewith.

The nucleotide sequence of a mutant of GDH1 is as shown in SEQ ID NO. 30, or a sequence having at least 70% homology therewith; the amino acid sequence of a protein formed after the expression of the mutant of GDH1 is shown in SEQ ID NO. 43, or a sequence having at least 70% homology therewith.

The nucleotide sequence of SuhB is as shown in SEQ ID NO. 23, or a sequence having at least 70% homology therewith; the amino acid sequence of a protein formed after the expression of SuhB is shown in SEQ ID NO. 44, or a sequence having at least 70% homology therewith.

In a third aspect, the present disclosure provides use of a strain for producing glucose and derivatives thereof in a high yield in the transformation of non-grain low-carbon carbon sources.

Glucose, sucrose, glucosamine, inositol, analogs or derivatives of glucose, analogs or derivatives of sucrose, analogs or derivatives of glucosamine, and analogs or derivatives of inositol are produced by means of biotransformation using any recombinant yeast strain described above.

Preferably, the analogs of glucose are isomers of glucose, and the derivatives of glucose include derivatives of alcohols, amines, oligosaccharides, and polysaccharides of glucose.

More preferably, the analogs of glucose include fructose and galactose, and the derivatives of glucose include sugar alcohol, ammonia sugar, disaccharide sucrose, and polysaccharide starch.

For the use, the recombinant yeast strain A or the recombinant yeast strain E is used to ferment a carbon source as a substrate in a culture medium to obtain glucose.

The recombinant yeast strain B or the recombinant yeast strain F is used to ferment a carbon source as a substrate in a culture medium to obtain glucosamine.

The recombinant yeast strain C or the recombinant yeast strain G is used to ferment a carbon source as a substrate in a culture medium to obtain sucrose.

The recombinant yeast strain D or the recombinant yeast strain H is used to ferment a carbon source as a substrate in a culture medium to obtain inositol.

Preferably, the carbon source is a non-grain carbon source, including acetic acid, methanol, ethanol, propanol, and glycerol.

The beneficial effects of the present disclosure are as follows: due to the characteristics that microorganisms use glucose, no industrial utilization of microbial fermentation to produce glucose/sucrose/glucosamine/inositol is implemented currently; while in the present disclosure, with a non-grain carbon source prepared by photoelectric catalysis or traditional chemical synthesis as a substrate, by means of a recombinant yeast strain, the quick preparation of food raw materials glucose/sucrose/glucosamine/inositol and then the production of food needed by human life can be achieved.

### BRIEF DESCRIPTION OF DRAWINGS

FIG. 1 is a schematic diagram of the construction of a recombinant *Saccharomyces cerevisiae* strain according to an example of the present disclosure;
FIG. 2 is a graph of the validation result of the glucose utilization deficiency of the recombinant *Saccharomyces cerevisiae* strain in Example 1 of the present disclosure;
FIG. 3 is a graph of the content detection result of glucose produced by means of biotransformation with the recombinant *Saccharomyces cerevisiae* strain in Example 1 of the present disclosure;
FIG. 4 is a graph of the content detection result of glucose produced by means of biotransformation with the recombinant *Saccharomyces cerevisiae* strain in Example 2 of the present disclosure;
FIG. 5 is a graph of the content detection result of glucose produced by means of fermentation with the LY031 using an electrocatalytically synthesized acetic acid as a substrate in Example 3 of the present disclosure;
FIG. 6 is a graph of the effect of glucose metabolic pathway knockout in Example 4 of the present disclosure;
FIG. 7 is a graph of the content detection result of glucose produced by means of biotransformation with a recombinant *Pichia pastoris*/*Saccharomyces cerevisiae* strain in Example 5 of the present disclosure;
FIG. 8 is a graph of the effect of sucrose metabolic pathway knockout in Example 8 of the present disclosure;
FIG. 9 is a graph of the result of biosynthesis and yield improvement of sucrose in Example 8 of the present disclosure;
FIG. 10 is a graph of the result of biosynthesis and yield improvement of ammonia sugar in Example 10 of the present disclosure; and
FIG. 11 is a graph of the result of biosynthesis and yield improvement of inositol in Example 12 of the present disclosure.

### DETAILED DESCRIPTION

For a better understanding of the content of the present disclosure, the content of the present disclosure will be further described below in conjunction with examples, but the content of the present disclosure is not limited to the examples set forth below.

In Example 1 to Example 12 of the present disclosure, *Saccharomyces cerevisiae* and *Pichia pastoris* are used as examples to describe in detail a method for producing glucose and derivatives thereof by means of biotransformation with a recombinant yeast, where the information about the *Saccharomyces cerevisiae* strain Lab001 used herein is: MATa *ura3-52 can1Δ::cas9-natNT2 TRPI LEU2 HIS3,* and the information of the *Pichia pastoris* strain GSY002 used herein is: *GS115: his4 Ku70Δ::Rad52.*

The *Saccharomyces cerevisiae* strains in the examples of the present disclosure are all constructed using a CRISPR/Cas9 method, with reference to the literature (Robert, M., Van, R. H. M., Melanie, W., et al. CRISPR/Cas9: a molecular Swiss army knife for the simultaneous introduction of multiple genetic modifications in Saccharomyces cerevisiae[J]. Fems Yeast Research, 2015, (2): 2). The *Saccharomyces cerevisiae* transformation method refers to the literature (Gietz, R. D., Woods, R. A., Transformation of yeast by the LiAc/ss carrier DNA/PEG method[J]. Methods in Molecular Biology, 2006, 313: 107-120). The *Pichia pastoris* strains are all constructed using a CRISPR/Cas9 method, with reference to the literature (Thomas, Gassler, Lina, et al. CRISPR/Cas9-Mediated Homology-Directed Genome Editing in Pichia pastoris[J]. Methods in Molecular Biology, 2019). The *Pichia pastoris* transformation method refers to the literature (Joan, L. C., Wong, W. W., Xiong, S., et al. Condensed protocol for competent cell preparation and transformation of the methylotrophic yeast Pichia pastoris[J]. Biotechniques, 2005, 38 (1): 48-0). Experimental methods without specific conditions noted in the following examples are conducted according to conventional conditions, that is, conditions described in Molecular Cloning: A Laboratory Manual (New York: Cold Spring Harbor Laboratory Press, 2001).

In the following examples, first, a glucokinase gene *glkl* (NCBI-GeneID: 850317) having a glucokinase activity and two hexokinase isoenzyme genes *hxkl* (NCBI-GeneID:850614) and *hxk2* (NCBI-GeneID: 852639) in *Saccharomyces cerevisiae* are knocked out to obtain a recombinant *Saccharomyces cerevisiae* strain with a glucose utilization deficiency and a capability to secrete glucose; then, putative hexokinase genes *emi2* (NCBI-GeneID: 852128) and *YLR446W* (NCBI-GeneID: 851167) in the recombinant *Saccharomyces cerevisiae* strain with a glucose utilization deficiency and a capability to secrete glucose are knocked out, and a glucose phosphatase gene *agpP* (the nucleotide sequence of the *agpP* gene is as shown in SEQ ID NO. 1) (the phosphatase possesses glucose-1-phosphate and glucose-6-phosphate activities) from *Pantoea* and a haloacid dehalogenase-like phosphatase 4 (HAD4) gene *yihx* (the nucleotide sequence of the *yihx* gene is as shown in SEQ ID NO. 2) (the enzyme produced after the encoding of the gene specifically hydrolyzes glucose-1-phosphate to produce glucose) from *Escherichia coli* are overexpressed at *YLR446W* and *emi2* sites, respectively, to effectively improve the secretion yield of glucose by 30%. Fermentation is performed using an electrocatalytically synthesized acetic acid as a carbon source, and the yield of glucose is up to 1.81 g/L.

The knockout method used in the experiment may also be other technologies that can achieve the same effect, such as RNA interference, enzyme activity reduction, and low-intensity promoter replacement or knockout.

### Example 1 Preparation of a recombinant Saccharomyces cerevisiae strain with a glucose utilization deficiency and a capability to secrete glucose

A strain with *glkl* knocked out was constructed through the following specific steps:
With Lab001 as the starting strain, a *glk1* knockout gRNA primers e1 and e2 were designed and obtained on a website (http://yeastriction.tnw.tudelft.nl/#!/), and the obtained primers were used to amplify a 2 µm fragment. The reaction system is shown in Table 1.

**Table 1 Reaction system**

| | |
|---|---|
| pROS10 plasmid template | 40 ng |
| gRNA primer 1 (10 µM) | 1 µL |
| gRNA primer 2 (10 µM) | 1 µL |
| 2× PrimeSTAR^{®} Max DNA Polymerase | 25 µL |
| Total volume (with ddH₂O added) | Add to 50 µL |

The plasmid skeleton was amplified using a primer e55. The reaction system is shown in Table 2.

**Table 2 Reaction system**

| | |
|---|---|
| pROS10 plasmid template | 40 ng |
| Primer 1 (10 µM) | 2 µL |
| 2× PrimeSTAR^{®} Max DNA Polymerase | 25 µL |
| Total volume (with ddHzO added) | Add to 50 µL |

The 2 µm fragment and the plasmid skeleton were assembled using a Gibson Assembly method to construct a *glkl* knockout plasmid. With a *Saccharomyces cerevisiae* genome as a template, a primer e9 and a primer e10 amplified a *glk1* knockout repair upstream fragment; a primer e11 and a primer e12 amplified a *glkl* knockout repair downstream fragment; after the fragments were obtained, fusion PCR was performed using the primer e9 and the primer e12 to obtain a repair fragment Glk1-UP-Glk1-DN. The specific steps are as follows: Fresh yeasts were cloned into 1 mL of YPE medium (yeast extract peptone ethanol medium) and cultured overnight. An appropriate amount of yeast liquid was collected and transferred into 20 mL of YPE, cultured at 30 °C from starting OD₆₀₀ = 0.1 to OD₆₀₀ = 0.6, and centrifuged at 4200 rpm to remove the medium. The cell precipitate was resuspended with 1 mL of sterile water and centrifuged at 4200 rpm to remove the supernatant. The cells were resuspended with 1 mL of 0.1 M lithium acetate and centrifuged at 4200 rpm to remove the supernatant, and 200 µL of 0.1 M lithium acetate was added to obtain competent cells of *Saccharomyces cerevisiae* cells. The recombinant *Saccharomyces cerevisiae* construction was performed on the yeast competent cells using a lithium acetate/polyethylene glycol transformation method, and the transformation system is shown in Table 3.

**Table 3 Transformation system**

| | |
|---|---|
| Polyethylene glycol 3500 (50% w/v) | 240 µL |
| Lithium acetate (1.0 M) | 36 µL |
| Salmon sperm DNA (2.0 mg/mL) | 25 µL |
| Repair fragment | 1-2 µg |
| Knockout plasmid | 1-2 µg |
| Total volume (with water added) | Add to 351 µL |

The above transformation liquid was mixed evenly, cultured at 30 °C for 30 minutes, and heat-shocked at 42 °C for 40 minutes. The heat-shocked transformation liquid was centrifuged at a speed of 5000 rpm for 1 minute to remove the supernatant. After the supernatant was removed, 100 µL of water was added to the transformation liquid, mixed evenly, coated on a solid medium of SC-URA + 2% (v/v) ethanol evenly, and finally cultured at 30 °C for 72 hours to obtain the recombinant strain LY021. LY021 was selected and cultured in a liquid medium of SC-URA + 2% ethanol (v/v) overnight. 200 µL of culture liquid was collected to extract a genome, and with the genome as the template, verification was performed using primers e13 and e14 to obtain the correct transformants.

At the same time, with Lab001 as the starting strain, an *hxkl* knockout plasmid was obtained using the primer combination of e3 and e4, *hxkl* upstream and downstream fragments were obtained using the primer combination of e15 and e16 and the primer combination of e17 and e18, an *hxkl* knockout repair fragment was obtained using e15 and e18, transformation was performed to obtain the transformers, and the transformers were verified using primers e19 and e20 to obtain LY022. With Lab001 as the starting strain, an *hxk2* knockout plasmid was obtained using the primer combination of e5 and e6, *hxk2* upstream and downstream fragments were obtained using the primer combination of e21 and e22 and the primer combination of e23 and e24, an *hxk2* knockout repair fragment was obtained using e21 and e24, transformation was performed to obtain the transformers, and the transformers were verified using primers e25 and e26 to obtain LY023.

With Lab001 as the starting strain, a *glk1-hxk1* knockout plasmid was obtained using the primer combination of e1 and e3, the *glk1-hxk1* knockout plasmid, a *glk1* knockout repair fragment, and the *hxkl* knockout repair fragment were transformed into transformants, and the transformants were verified using primers e13, e14, e19, and e20 to obtain a *glk1-hxk1* double knockout strain LY024.

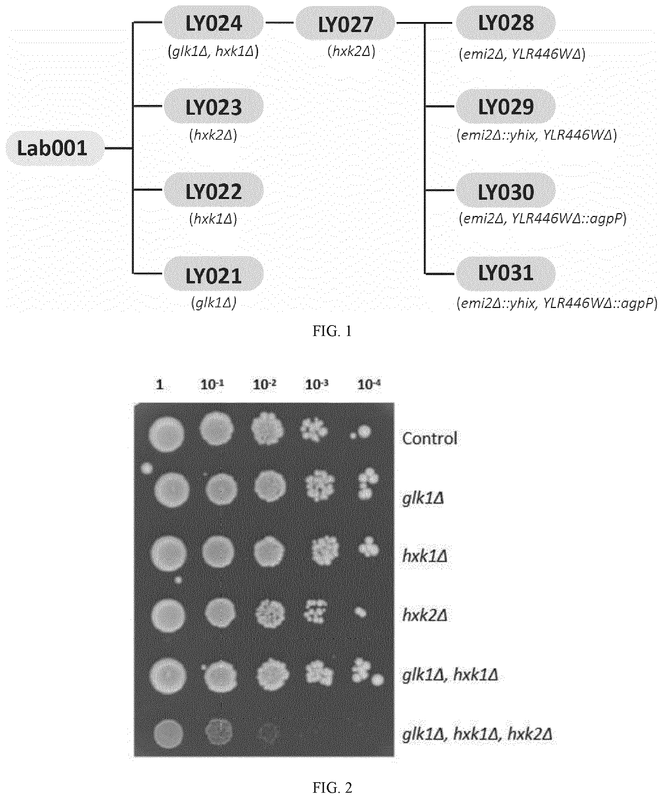

With LY024 as the starting strain, *hxk2* was knocked out, and construction was performed with reference to LY023 to obtain LY027. The strain construction is shown in FIG. 1.

To verify the glucose utilization capability of the engineered strains, the starting strains and the engineered strains were tested on the dot plate separately. The specific steps are as follows: The SC agar plate with glucose as a single carbon source was configured, fresh yeast cells were diluted by serial dilution to OD₆₀₀ = 1, 0.1, 0.01, 0.001, and 0.0001, respectively, and 5 µL of diluted yeast cells were collected for dot plate test. The test result is shown in FIG. 2. As shown in the figure, with the deletion of *glk1, hxk1,* and *hxk2,* the engineered strains had a glucose utilization deficiency.

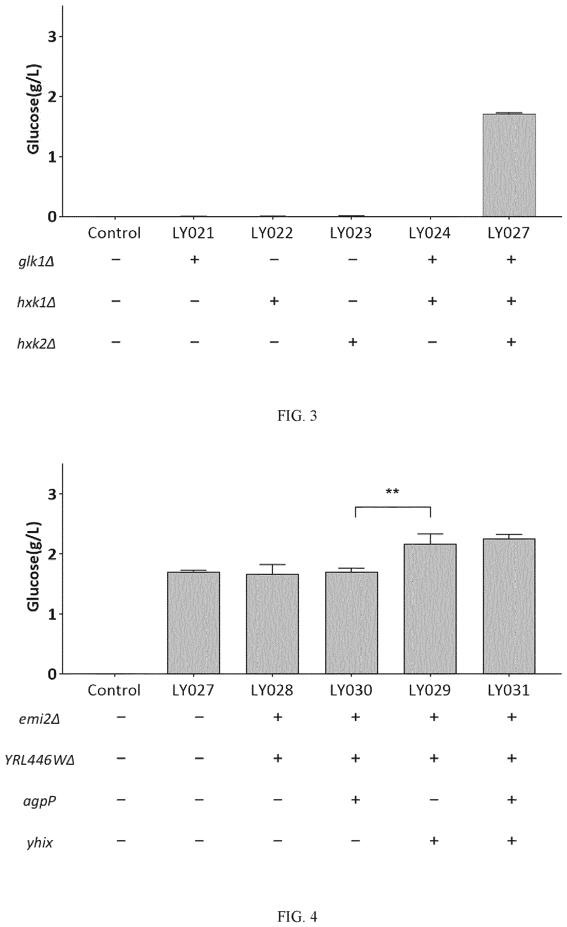

At the same time, the generation of glucose was analyzed by high-performance liquid chromatography (HPLC). The specific steps are as follows: fresh yeast cells were inoculated to a Delft medium (Minimal medium). Referring to the literature (Verduyn, C., Postma, E., Scheffers, W. A., Van Dijken, J. P., 1992. Effect of benzoic acid on metabolic fluxes in yeasts: A continuous-culture study on the regulation of respiration and alcoholic fermentation. Yeast, 8: 501-517. doi: 10.1002/yea.320080703), with ethanol as the sole carbon source and initial OD₆₀₀ = 0.1, 1 mL of fermentation liquid was collected after 6 days of fermentation and centrifuged at 12000 rpm for 10 minutes, and the supernatant was filtered through a 0.22 µm filter membrane and analyzed by HPLC. The HPLC is as follows: the column was Aminex HPX-87H; the mobile phase was 5 mM H₂SO₄; the column temperature was 50 °C; the injection volume was 5 µL; the test duration of each sample was 30 minutes; the glucose detector was a differential detector. The test result is shown in FIG. 3. The production of glucose was detected in the fermentation liquid of the engineered strain with the deletion of all *glk1, hxk1* and *hxk2,* and the content was 1.7 g/L.

### Example 2 Preparation of a recombinant Saccharomyces cerevisiae strain with an improved glucose synthesis yield

With LY027 as the starting strain to construct an *emi2* and *YLR446W* double-deletion strain, *emi2* and *YLR446W* knockout gRNA primers e7 and e8 were designed on the website (http://yeastriction.tnw.tudelft.nl/#!/) to obtain the primers to amplify a 2 µm fragment, and with reference to Example 1, an *emi2* and *YLR446W* double-knockout plasmid was constructed. With a *Saccharomyces cerevisiae* genome as the template, an emi2 UP-emi2 DN repair fragment was constructed using primers e41 and e42 and primers e51 and e52, respectively; a YLR446W-UP-YLR446W-DN repair fragment was constructed using primers e27 and e28 and primers e36 and e38; a YLR446W UP-CCW12p-agpP-PYK1t-YLR446W DN repair fragment was constructed using primers e27 and e29, primers e30 and e31, primers e32 and e33, primers e34 and e35, and primers e37 and e38, where CCW12p was the *Saccharomyces cerevisiae* CCW12 promoter (SEQ ID NO. 3), and PYKlt was the *Saccharomyces cerevisiae* PYK1 terminator (SEQ ID NO. 4); an EMI2 UP-TEF1p-yihx-DIT1t-EMI2 DN repair fragment was constructed using primers e41 and e43, primers e44 and e45, primers e46 and e47, primers e48 and e49, and primers e50 and e52, where TEFlp was the *Saccharomyces cerevisiae* TEF1 promoter (SEQ ID NO. 5), and DIT1t was the *Saccharomyces cerevisiae* DIT1 terminator (SEQ ID NO. 6). With reference to Example 1, with the engineered strain LY027 as the starting strain and using primers e39 and e40 and primers e53 and e54 for identification, the engineered strain LY028, LY029, LY030, and LY031 were constructed and obtained, and their strain genotypes are shown in FIG. 1; fermentation analysis was performed on the obtained *Saccharomyces cerevisiae* strains, where for fermentation analysis, reference was made to Example 1. The results are shown in FIG. 4. After the knockout of the two putative hexokinase genes in *Saccharomyces cerevisiae* and the knockout of the two putative hexokinase genes, the introduction of the glucose phosphatase gene *agpP* from *Pantoea* alone did not significantly improve the glucose yield; whereas the introduction of the HAD4 gene *yihx* from *Escherichia coli* significantly improved the glucose yield, and the glucose yield reached 2.2 g/L in the strain LY031, which was a 30% increase in yield. The sequences of the primers e1 to e55 used by *Saccharomyces cerevisiae* to produce glucose in Examples 1 to 2 of the present disclosure are shown in Table 4.

**Table 4 Sequences of the primers e1 to e55 used by Saccharomyces cerevisiae to produce glucose in Examples 1 to 2 of the present disclosure**

| | | |
|---|---|---|
| e1 | GLK1-gRNA1 | CGCAGTGAAAGATAAATGATCTGCTTACTTCATCGAACAAAG TTTTAGAGCTAGAAATAGCAAGT |
| e2 | GLK1-gRNA2 | CGCAGTGAAAGATAAATGATCATGATCATCAATGTCGAATGGT TTTAGAGCTAGAAATAGCAAGT |
| e3 | HXK1-gRNA1 | CGCAGTGAAAGATAAATGATCAGAAGACTTTGTGAATTGATG TTTTAGAGCTAGAAATAGCAAGT |
| e4 | HXK1-gRNA2 | CGCAGTGAAAGATAAATGATCAATAAAGGTTTGACAAAGAA GTTTTAGAGCTAGAAATAGCAAGT |
| e5 | HXK2-gRNA1 | CGCAGTGAAAGATAAATGATCACATCGTGGTTTTCAATGTTGT TTTAGAGCTAGAAATAGCAAGT |
| e6 | HXK2-gRNA2 | CGCAGTGAAAGATAAATGATCAATGCTTTGAAGGACATTTAG TTTTAGAGCTAGAAATAGCAAGT |
| e7 | EMI2-gRNA1 | CGCAGTGAAAGATAAATGATCTTGTCATTTAGATATTCTTCGT TTTAGAGCTAGAAATAGCAAGT |
| e8 | YLR446W-RNA1 | CGCAGTGAAAGATAAATGATCGATTCCATCTCATTAATAATGT TTTAGAGCTAGAAATAGCAAGT |
| e9 | Glk1-UP-F | GTATGTCCATACGGGTTTTTCG |
| e10 | Glk1-UP-R | CTTGTGTATGATAGAGTTGTATTAGTGGTG |
| e11 | Glk1--DN-F | CACCACTAATACAACTCTATCATACACAAGTCTTTTTACATTTT TTTGGTTTGTGTACG |
| e12 | Glk1-DN-R | GCAAACAATCTGCCAATTAATAAGATG |
| e13 | Glk1-VF | GAAAACTTCATAACGGGATAGATGTATG |
| e14 | Glk1-VR | CTTTGAAGCCATTCGGACCTTAG |
| e15 | HXK1-UP-F | GTACGTGTTCCTGTCGTTTATC |
| e16 | HXK1-UP-R | CTTATTTTTTCAGTATTCTAATTGAGTTG |
| e17 | HXK1-DN-F | AATTAGAATACTGAAAAAATAAGTGAAAAAAATGTAATGAAA TATAAATGTGTTTTTCC |
| e18 | HXK1-DN-R | CAGAATTCGACTCTTTTACCGATATC |
| e19 | HXK1-VF | GGTTGGAAATAAACCGGAAAAATG |
| e20 | HXK1-VR | CCAGAAAGCATGGGATCTTCT |
| e21 | HXK2-UP-F | GAGTTTTCTGAACCTCCTCGC |
| e22 | HXK2-UP-R | TTTATTTAATTAACGTACTTATTATGTGTGGAGAATTATA |
| e23 | HXK2-DN-F | CATAATAAGTACGTTAATTAAATAAAACTTAATTTGTAAATTAA GTTTGAACAACAAG |
| e24 | HXK2-DN-R | GGTAAAAGAAGAATCCACGCG |
| e25 | HXK2-VF | CCAGAGTATACTGCTCTTTCTAATG |
| e26 | HXK2-VR | GTTTCTAAGCGTAGTGAGGTG |
| e27 | YLR446W-UP-F | GCAAATTTAATAACAGCAACAACG |
| e28 | YLR446W(dn )-UP-R | CGAGAAGATGGTAAGAAATGATGAAATATAGTTCTGAACCTC CTTTTGTTAAC |
| e29 | YLR446W(C CW12p)-UP-R | CTTTTATTTTGCTTTGCCCTGGTTGTTCTGAACCTCCTTTTGTT AAC |
| e30 | CCW12p-F | AACCAGGGCAAAGCAAAATAAAAG |
| e31 | CCW12p-R | TATTGATATAGTGTTTAAGCGAATGACAG |
| e32 | agpP(CCW12 p)-F | CTGTCATTCGCTTAAACACTATATCAATAATGATTAAGAAGTTA TCTTTGTGTGC |
| e33 | agpP(PYK1t)-R | AATATCTTCATTCAATCATGATTCTTTTTTATTGAGCAGCAGT AGCAACAG |
| e34 | PYK1t-F | AAAAAGAATCATGATTGAATGAAGATATTATTTTTTTG |
| e35 | PYK1t-R | GCATTTATGTACCCATGTATAACCTTC |
| e36 | YLR446W(up )-DN-F | GTTAACAAAAGGAGGTTCAGAACTATATTTCATCATTTCTTAC CATCTTCTCG |
| e37 | YLR446W(P YK1t)-DN-F | GAAGGTTATACATGGGTACATAAATGCTATATTTCATCATTTCT TACCATCTTCTCG |
| e38 | YLR446W-DN-R | CACTTTTACCAATTGGAAATGGAAC |
| e39 | YLR446W-VF | CTGTTCCTGTAAACATTATTGAGCA |
| e40 | YLR446W-VR | CTGCTTTAGCTAACGTCTATGAG |
| e41 | EMI2-UP-F | GATGCTATCTTCAAAATGGCTGTC |
| e42 | EMI2(DN)-UP-R | GCTGTTAAGAACAGATATGTTTACTGTGTATCTATGATTCGATT CTTGTAC |
| e43 | EMI2(TEF1p) -UP-R | GTAAAAAAGGAGTAGAAACATTTTGAAGCTATTACTGTGTAT CTATGATTCGATTCTTG |
| e44 | TEF1p-F | ATAGCTTCAAAATGTTTCTACTCCTTTTTTAC |
| e45 | TEF1p-R | TTTGTAATTAAAACTTAGATTAGATTGCTATGC |
| e46 | Yihx(TEF1P)-F | GCATAGCAATCTAATCTAAGTTTTAATTACAAAATGTTGTACAT TTTCGATTTGGG |
| e47 | Yihx(DIT1t)-R | GACCAATGTAGCGCTCTTACTTTATTAACACAAAACCTTAGCG AAGTAATC |
| e48 | DIT1t-F | TAAAGTAAGAGCGCTACATTGGTC |
| e49 | DIT1t-R | GTTACTCCGCAACGCTTTTC |
| e50 | EMI2(DIT1t)-dn-F | GAAAAGCGTTGCGGAGTAACAACATATCTGTTCTTAACAGCT TATTTTTAG |
| e51 | EMI2(UP)-dn-F | GAATCGAATCATAGATACACAGTAAACATATCTGTTCTTAACA GCTTATTTTTAG |
| e52 | EMI2-DN-R | CTTGGAAGCATAGAAGGTGCTC |
| e53 | EMI2-VF | GCCAAAATGCAACTAAACCTCG |
| e54 | EMI2-VR | GGTTAATTTATCTCTTGGTGGAGACC |
| e55 | Primer 1 | GATCATTTATCTTTCACTGCGGAGAAG |

### Example 3 Method for producing glucose by means of biotransformation with an electrocatalytically synthesized acetic acid and a recombinant Saccharomyces cerevisiae strain

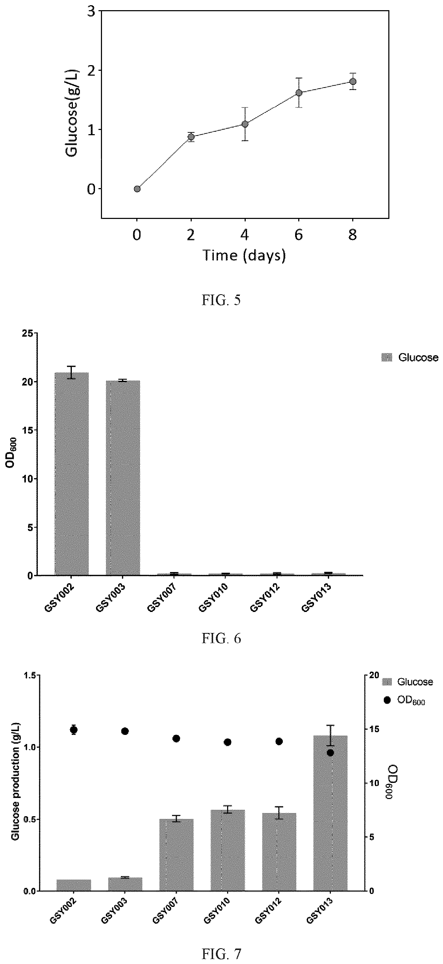

With an electrocatalytically synthesized acetic acid as a carbon source to synthesize glucose by means of biotransformation, the recombinant *Saccharomyces cerevisiae* strain (LY031) overexpressing both the glucose phosphatase gene *agpP* from *Pantoea* and the HAD4 (haloacid dehalogenase-like phosphatase 4) gene *yihx* from *Escherichia coli ,* prepared in Example 2, was streaked on a fresh YPE solid plate and cultured at 30 °C for 5 days; a single colony was transferred to 1 mL of YPE liquid medium and cultured at 30 °C and 200 rpm until OD₆₀₀ = 8 to obtain a culture liquid; the culture liquid was centrifuged at 4200 rpm to remove the supernatant and centrifuged with 1 mL of sterile water added to remove the supernatant, and this step was repeated one more time; the culture liquid was resuspended with 1 mL of sterile water, then transferred to a shake flask with 20 mL of Delft E (2% v/v), and cultured at 30 °C for four days; all the yeast liquid was transferred to a 50 mL centrifuge tube, centrifuged to remove the supernatant, resuspended with 20 mL of sterile water, and centrifuged to remove the supernatant, and this step was repeated one more time. The remaining cells were resuspended using 20 mL of Delft medium with acetic acid (1% by mass, electrocatalytically synthesized) as a carbon source, transferred to a shake flask, and cultured at 30 °C and 200 rpm. 400 µL of fermentation liquid were collected every two days for glucose content detection, then acetic acid (electrocatalytically synthesized) was added on the second and the fourth day until the final concentration reached 1%, and the cells were further cultured. The glucose content detection is shown in FIG. 5, the total amount of acetic acid added was 29.52 g/L, and the glucose yield was 1.81 g/L.

### Example 4 Preparation of a recombinant Pichia pastoris strain with a glucose utilization deficiency and a capability to secrete glucose

A plasmid containing gRNA was constructed: the plasmid BB3cH_pGAP_23_pLAT1_Cas9 was digested using a restriction incision enzyme. The digestion system is shown in Table 5.

**Table 5 Digestion system**

| | |
|---|---|
| BB3cH_pGAP_23_pLAT1_Cas9 | 3 µg |
| 10× CutSmart Buffer | 5 µL |
| BbsI | 1 µL (10 U) |
| ddH₂O | Add to 50 µL |

A gRNA plasmid skeleton was obtained by digestion in water bath at 37 °C for 3 hours. PCR amplification was performed using primers d1, d2, d9, d10, d11, and d12 according to the following system in Table 6: pre-denaturation at 98 °C for 30 seconds, denaturation at 98 °C for 15 seconds, annealing at 60 °C for 20 seconds, extension at 72 °C for 10 seconds, and finally, total extension at 72 °C for 2 minutes to obtain a Glk1-gRNAfragment, where 35 cycles were set from denaturation to extension.

**Table 6 gRNA fragment system**

| | |
|---|---|
| GLK1_1_sgRNA_F (10 µM) | 2.5 µL |
| B_sgRNA_stru_Rw (10 µM) | 2.5 µL |
| GLK1_2_sgRNA_F (1µ M) | 0.5 µL |
| A_sgRNA_stru_Rw (1µ M) | 0.5 µL |
| C_sgRNA_stru_Rw (1µ M) | 0.5 µL |
| D_sgRNA_stru_Fw (1µ M) | 0.5 µL |
| 10 mM dNTPs | 1 µL |
| 5× Buffer | 10 µL |
| Pusion | 0.5 µL |
| ddH₂O | Add to 50 µL |

The obtained plasmid skeleton and the Glk1-gRAN fragment were assembled in the Golden Gate mode. The assembly system is shown in Table 7. PCR amplification was performed at 37 °C for 2 minutes, at 16 °C for 2 minutes and 30 seconds, where the two steps were set for 30 cycles; and then performed at 37 °C for 10 minutes, at 55 °C for 30 minutes, and at 80 °C for 10 minutes. The DH5α competent cells were added to 10 µL of the system after the reaction, placed on ice for 30 minutes, heat-shocked at 42 °C for 90 seconds, then transferred onto ice for 5 minutes, and, with 1 mL of LB medium added, incubated in a shaking table with 180 rpm at 37 °C for 1 hour. The cells were centrifuged to remove the supernatant, and with 100 µL of sterile water added, coated on an LB solid plate containing 200 mg/L hygromycin. After 16 to 18 hours of culture, sequencing and plasmid extraction were performed on a single colony to obtain a Glk1-gRNA plasmid.

**Table 7 gRNA fragment system**

| | |
|---|---|
| BbsI (10 U) | 1 µL |
| T4 ligase (40 U) | 0.1 µL |
| 10× CutSmart^{®} Buffer | 2 µL |
| ATP (10mM) | 0.5 µL |
| Glk1-gRNA fragment | 10 ng |
| Plasmid skeleton | 5 ng |
| ddH₂O | Add to 20 µL |

Homologous fragment preparation: with *Pichia pastoris* genomic DNA as the template, PCR amplification was performed using primers d19 and d20, and primers d21 and d22, respectively, to obtain an upstream fragment Glk1-up and a downstream fragment Glk1-down with a Glk1 gene knocked out, and with Glk1-up and Glk1-down as the template, PCR amplification was performed using primers d19 and d22 to obtain a repair fragment Glk1-fragment.

Gene knockout and validation: A single colony of *Pichiapastoris* strain GSY002 was added to 2 mL of YPG (2% glycerol) liquid medium and cultured in a shaking table at 30 °C and at a speed of 200 rpm overnight. The activated strain was transferred into 50 mL of YPG liquid medium to make the starting OD₆₀₀ = 0.2, and then cultured in a shaking table at 30 °C and at a speed of 200 rpm for 4 to 6 hours to make its OD₆₀₀ = 0.6 to 0.8. The strain was centrifuged at a speed of 3000 rpm for 5 minutes to remove the supernatant. 40 mL of pure water was added, and the strain was centrifuged at a speed of 3000 rpm for 5 minutes to remove the supernatant. 9 mL of pre-cooled BEDS (10 mM bicine-NaOH at pH of 8.3 + 3% (v/v) ethylene glycol + 5% (v/v) dimethyl sulfoxide + 1 M sorbitol) solution and 1 mL of 1.0 M DTT were added, and the strain was incubated in a shaking table at 30 °C and at a speed of 200 rpm for 5 minutes. The strain was centrifuged at a speed of 3000 rpm for 5 minutes to remove the supernatant, and then 1 mL of BEDS solution was added to prepare *Pichia pastoris* competent cells. 40 µL of competent cells, 1 µg of Glk1-gRAN plasmid, and 1 µg of Glk1-fragment were mixed, transferred to a 0.2 cm electroporation cuvette aseptically treated, placed on ice for 2 minutes, electrically shocked at 1.5 kV for 5 milliseconds. After the electric shock, 0.5 mL of 1.0 M sorbitol and 0.5 mL of YPG medium were added immediately, and then incubated in a shaking table at 30 °C and at a speed of 200 rpm for 1 to 3 hours, centrifuged to remove the supernatant, resuspended with 100 µL of sterile water, coated on a YPG solid plate containing 200 µg/L hygromycin, and cultured at 30 °C for 2 to 3 days. The monoclonal transformants were collected and cultured in a YPG liquid medium containing 200 µg/L hygromycin overnight. 200 µL of yeast liquid was collected to extract a genome, and with the genome as the template, and verification was performed using primers d23 and d24 to obtain a recombinant yeast strain GSY003 with the Glk1 gene knocked out.

On the basis of the above, with GSY003 as the starting strain, an Hxk1-gRNA fragment was obtained using the primer combination of d5, d6, d9, d10, d11, and d12, and with reference to the construction of the Glk1-gRNA plasmid, an Hxk1-gRNA plasmid was obtained. An Hxk1-fragment was obtained using primers d13 and d14 and primers d15 and d16, and verification was performed using primers d17 and d18 to finally obtain a recombinant yeast strain GSY007 with Hxk1 knocked out. With GSY007 as the starting strain, an Hxk2-gRNA fragment was obtained using the primer combination of d7, d8, d9, d10, d11, and d12, and with reference to the construction of the Glk1-gRNA plasmid, an Hxk2-gRNA plasmid was obtained. An Hxk2-fragment was obtained using primers d25 and d26 and primers d27 and d28, and verification was performed using primers d29 and d30 to finally obtain a recombinant yeast strain GSY010 with Hxk2 knocked out.

### Example 5 Preparation and use of a recombinant Pichia pastoris strain with an improved glucose synthesis yield

(1) Construction of a recombinant *Pichia pastoris* for producing glucose in a high yield: With GSY010 as the starting strain, an Hxk iso2-gRNA fragment was obtained using the primer combination of d3, d4, d9, d10, d11, and d12, and with reference to the construction of the Hxk iso2-gRNA plasmid, an Hxk iso2-gRNA plasmid was obtained; an Hxk iso2-fragment was obtained using primers d31 and d32 and primers d33 and d34, and verification was performed using primers d45 and d46 to finally obtain a recombinant yeast strain GSY012 with Hxk iso2 knocked out. Similarly, with GSY010 as the starting strain, five assembled fragments were obtained using primers d35 and d36, primers d37 and d38, primers d39 and d40, primers d41 and d42, and primers d43 and d44, an insert fragment Hxk iso2-fragment-2 (SEQ ID NO. 31) containing *yihx* at the Hxk iso2 site was finally obtained using primers d35 and d38, and verification was performed using primers d45 and d46 to finally obtain a recombinant *Pichia pastoris* strain GSY013.
(2) Recombinant *Pichia pastoris* glucose test: GSY002, GSY003, GSY007, GSY010, GSY012, and GSY013 were activated overnight with YPG, respectively. The activated strains were each cleaned twice with sterile water, inoculated in 20 mL of Delft D (2% glucose), with starting OD₆₀₀ = 0.1, and cultured at 30 °C and 200 rpm for 96 hours. Samples were collected to measure OD₆₀₀. The results are shown in FIG. 6: GSY002 and GSY003 could still grow in the glucose, while GSY007, GSY010, GSY012, and GSY013 cannot grow and lost the glucose utilization capability.
(3) Use of the recombinant *Pichia pastoris* in the production of glucose: GSY002, GSY003, GSY007, GSY010, GSY012, and GSY013 were selected and then activated overnight with YPG, respectively. The activated strains were each inoculated in 20 mL of Delft M (2% methanol), with starting OD₆₀₀ = 0.1, and cultured at 30 °C and 200 rpm for 96 hours. Samples were collected to measure OD₆₀₀ and subjected to glucose analysis, where for the analysis method, reference was made to Example 1. The results are shown in FIG. 7: GSY002 and GSY003 cannot produce glucose; GSY007, GSY010, and GSY012 could produce 0.5g/L glucose, and when *yihx* was overexpressed, the glucose yield reached 1.08 g/L, which was a 100% increase in yield, thereby effectively transforming a low-carbon non-grain carbon source methanol into glucose. The sequences of the primers d1 to d44 used by *Pichia pastoris* to produce glucose in Examples 4 to 5 of the present disclosure are shown in Table 8.

**Table 8 Sequences of the primers d1 to d44 used by Pichia pastoris to produce glucose in Examples 4 to 5 of the present disclosure**

| | | |
|---|---|---|
| d1 | GLK1_1_sgR NA_F | TGAAGACGCCATGAGTTCCCTGATGAGTCCGTGAGGACGAAA CGAGTAAGCTCGTCGGAA |
| d2 | GLK1_2_sgR NA_F | AAACGAGTAAGCTCGTCGGAACTTGGGTCACGTAGGCGTTTT AGAGCTAGAAATAGCAAG |
| d3 | HXK iso2_1_sgRN A_F | TGAAGACGCCATGTATGAGCTGATGAGTCCGTGAGGACGAAA CGAGTAAGCTCGTCCTCA |
| d4 | HXK iso2_2_sgRN A F | AAACGAGTAAGCTCGTCCTCATAAAGATTGCTCGTTAGTTTTA GAGCTAGAAATAGCAAG |
| d5 | HXKI_I_sgR NA_F | TGAAGACGCCATGCTTTCTCTGATGAGTCCGTGAGGACGAAA CGAGTAAGCTCGTCAGAA |
| d6 | HXK1_2_sgR NA F | AAACGAGTAAGCTCGTCAGAAAGGTGTTGGCTAGTTAGTTTT AGAGCTAGAAATAGCAAG |
| d7 | HXK2_1_sgR NA_F | TGAAGACGCCATGGGATGTCTGATGAGTCCGTGAGGACGAAA CGAGTAAGCTCGTCACAT |
| d8 | HXK2 _2_sgR NA F | AAACGAGTAAGCTCGTCACATCCCAATGCTACCTACCGTTTTA GAGCTAGAAATAGCAAG |
| d9 | D_sgRNA_str u Fw | GTTTTAGAGCTAGAAATAGCAAGTTAAAATAAGGCTAGTCCGT TATCAACTTGAAAAAGT |
| d1 0 | A_sgRNA_str u_Rw | GGCCGGCATGGTCCCAGCCTCCTCGCTGGCGCCGGCTGGGCA ACATGCTTCGGCATGGCG |
| d1 1 | B_sgRNA str u_Rw | AGAAGACGCAAGCAGTCCAAAGCTGTCCCATTCGCCATGCCG AAGCATGTTGCCCAGCCG |
| d1 2 | C_sgRNA_str u_Rw | AGGCTGGGACCATGCCGGCCAAAAGCACCGACTCGGTGCCA CTTTTTCAAGTTGATAACG |
| d1 3 | HXK1 dn-F | TTTATGTTTAGAGCTAGAGAATAAGGG |
| d1 4 | HXK1 dn-R | ATGTTAAATGTTTTACTCTACTTCCTTTC |
| d1 5 | HXK1 up-R (HXK1 dn) | GAAAGGAAGTAGAGTAAAACATTTAACATTATGGTGTGATGA AGTAGCAGAAGCTCTAG |
| d1 6 | HXK1 up-F | TTTAGACCTAATCTTTTTCTCTCTGATCCTC |
| d1 7 | HXK1 VF | ACCTTTGATAATGGTTGCCCCAG |
| d1 8 | HXK1 VR | CGAAATCCAACGTAGGCCTTCC |
| d1 9 | GLK1 up-F | TTTGCTGGAGAGTCTCCCACTAATTCATG |
| d2 0 | GLK1 up-R | AATAATTGTAGATTTCGGGGAGAAGAGG |
| d2 1 | GLK1 dn-F (GLK1 up) | CCTCTTCTCCCCGAAATCTACAATTATTGTACCCTTACTATTAC GTAATATGTATG |
| d2 2 | GLK dn-R | TTCTTGCCAAATTCATTTGAAAGCTCTTC |
| d2 3 | GLK1 VF | GTCTCTTGCTAAGTATGAGCTTATTG |
| d2 4 | GLK1 VR | CAGAGAATCTTAATGATGCTGTCAC |
| d2 5 | HXK2 up-F | CTGAATGACTTTGTCAACCAATGCAGCG |
| d2 6 | HXK2 up-R | TGGGATGTTAGAGCTGCTTGATTTCAAGCC |
| d2 7 | HXK2 dn-F (HXK2 up) | GAAATCAAGCAGCTCTAACATCCCATCCCAGATCTGCTTATGT AACGAAATATTTAC |
| d2 8 | HXK2 dn-R | ACATCACACATCTTTTCTCTATTCTTG |
| d2 9 | HXK2VF | GGAGCTAAATCGGTTGCCTTTCCAAGTG |
| d3 0 | HXK2 VR | CGAACTGTTTCGAAATCAGCTGTTC |
| d3 1 | HXK iso2 up-F | GGCAAACTTGAATAATGTCGACAATATG |
| d3 2 | HXK iso2 up-R | GTTCGTTCTAATCTTAAGTTCTTTACTTAAG |
| d3 3 | HXK iso2 dn-F (iso2 up) | GTAAAGAACTTAAGATTAGAACGAACTTAGATTAGTTAGTATG CATCGCGCGATCAG |
| d3 4 | HXK iso2 dn-R | GCTTAATAATATTATTCAATATAACATCTTGTATG |
| d3 5 | HXK iso2 up-F-2 | GGCAAACTTGAATAATGTCGACAATATG |
| d3 6 | HXK iso2 up_R (PET9p) | CAGTGGTGAATTTTCTAGGGCCCGTACTGTTCGTTCTAATCTT AAGTTCTTTACTTAAG |
| d3 7 | HXK iso2-dn-F-2 (DAS1t) | CACTGATGATTACAATTTGGTTAGATTAGTTAGTATGCATCG |
| d3 8 | HXK iso2-dn-R-2 | GCTTAATAATATTATTCAATATAACATCTTGTATGATTC |
| d3 9 | PpDAS1t-R | CCAAATTGTAATCATCAGTGATTATG |
| d4 0 | PpDAS1t-F | ACGGGAAGTCTTTACAGTTTTAGTTAG |
| d4 1 | Yihx-R (PpDAS1t) | TAACTAAAACTGTAAAGACTTCCCGTTTAACACAAAACCTTA GCGAAGTAATCTGG |
| d4 2 | Yihx-F (PpPET9p) | CAACAACAAGTCTAACTTCTTCGTCGACTTCATGTTGTACATT TTCGATTTGGGTAAC |
| d4 3 | PpPET9p-R | GAAGTCGACGAAGAAGTTAGACTTGTTG |
| d4 4 | PpPET9p-F | AGTACGGGCCCTAGAAAATTCACCACTG |
| d4 5 | HXK iso2-VF | CGATTTACTCAATAAGGTGGCACC |
| d4 6 | HXK iso2-VR | GGTTATACATCTTGAGAATGACTCTG |

### Example 6 Construction of a recombinant strain with a sucrose metabolic pathway gene knocked out

Construction of a plasmid containing gRNA: With pROS10 as the template, PCR amplification was performed using a primer 1 to obtain a plasmid skeleton, where the conditions of PCR amplification were: using 2× Phanta^{®} Max Master Mix enzyme, pre-denaturation was performed at 95 °C for 30 seconds, denaturation was performed at 95 °C for 15 seconds, annealing was performed at 56 °C for 15 seconds, extension was performed at 72 °C for 3 seconds, and finally, total extension was performed at 72 °C for 5 minutes, where 35 cycles were set from denaturation to extension. With pROS 1 0 as the template, PCR amplification was performed using primers 2 and 3, primers 9 and 10, and primers 23 and 24 to obtain 2 µm fragments containing SUC2-gRNA (SUC2-gRNA1/SUC2-gRNA2), MAL-gRNA/IMA-gRNA, and IMA5 gRNA (IMA5 gRNA1/IMA5 gRNA2), respectively, where the conditions of PCR amplification were: using 2× Phanta^{®} Max Master Mix enzyme, pre-denaturation was performed at 95 °C for 30 seconds, denaturation was performed at 95 °C for 15 seconds, annealing was performed at 56 °C for 15 seconds, extension was performed at 72 °C for 30 seconds, and finally, total extension was performed at 72 °C for 5 minutes, where 35 cycles were set from denaturation to extension. Each of 2 µm fragments containing SUC2-gRNA (SUC2-gRNA1/SUC2-gRNA2), MAL-gRNA/IMA-gRNA, and IMA5 gRNA (IMA5 gRNA1/IMA5 gRNA2) and the plasmid skeleton were added to a Gibson reaction liquid in an equal molar ratio, incubated at 50 °C for 1 hour, and then used to transform *Escherichia coli* competent cells, and the transformed *Escherichia coli* was coated on an LB + amp plate and grew overnight. The plasmids were extracted from grown colonies and sequenced to obtain SUC2-gRNA, MAL/IMA-gRNA, and IMA5-gRNA, respectively.

Homologous fragment preparation: With *Saccharomyces cerevisiae* genomic DNA as the template, PCR amplification was performed using primers 4 and 5 and primers 6 and 7, respectively, to obtain an upstream fragment SUC-up and a downstream fragment SUC-down with an SUC2 gene knocked out, and with SUC-up and SUC-down as the template, PCR amplification was performed using primers 4 and 7 to obtain a repair fragment SUC2-fragment, where the conditions of PCR amplification were: using 2× Phanta^{®} Max Master Mix enzyme, pre-denaturation was performed at 95 °C for 30 seconds, denaturation was performed at 95 °C for 15 seconds, annealing was performed at 56 °C for 15 seconds, extension was performed at 72 °C for 30 seconds, and finally, total extension was performed at 72 °C for 5 minutes, where 35 cycles were set from denaturation to extension. Similarly, PCR amplification was performed using primers 25 and 26 and primers 27 and 28 to obtain an IMA5-fragment. Annealing was performed using primers 11, 12, 13, and 14 to form an IMA-fragment, and annealing was performed using primers 16, 17, 18, and 19 to form an MAL-fragment.

Gene knockout and validation: A single colony of a wide yeast strain was added to 2 mL of YPD liquid medium and cultured in a shaking table at 30 °C and at a speed of 250 rpm for 12 hours to activate the strain. The activated strain was transferred into 40 mL of YPD liquid medium to make the starting OD₆₀₀ = 0.2, and then cultured in a shaking table at 30 °C and at a speed of 200 rpm for 4 to 6 hours to make its OD₆₀₀ = 0.6 to 1.0. The strain was centrifuged at a speed of 3000 rpm for 5 minutes to remove the supernatant. 40 mL of pure water was added, and the strain was centrifuged at a speed of 3000 rpm for 5 minutes to remove the supernatant. 20 mL of lithium acetate at a concentration of 100 mM was added to the strain, mixed evenly, and centrifuged at a speed of 3000 rpm for 5 minutes to remove the supernatant. The cells were resuspended with 400 µL of lithium acetate at a concentration of 100 mM to prepare *Saccharomyces cerevisiae* competent cells. 50 µL of prepared *Saccharomyces cerevisiae* competent cells were subpackaged into a 1.5 mL centrifuge tube, 240 µL of PEG3350 at a concentration of 50% (W/V), 36 µL of lithium acetate at a concentration of 1M at, 5µL of single-stranded fish sperm DNA (which was, before use, cooked at 99 °C for 5 to 10 minutes and then placed immediately on ice) at a concentration of 10 mg/mL, 500 ng of recombinant plasmid SUC2-gRNA, and 1000 ng of repair fragment were added, and the above transformation liquid was mixed evenly. After mixing evenly, the transformation liquid was cultured at 30 °C for 30 minutes and heat-shocked at 42 °C for 40 minutes. The heat-shocked transformation liquid was centrifuged at a speed of 5000 rpm for 1 minute to remove the supernatant. After the supernatant was removed, 100 µL of water was added, mixed evenly, coated on an SC-URA solid medium evenly, and cultured at 30 °C for 72 hours to obtain a recombinant strain AT01.

ATOP was selected and cultured in a liquid medium of SC-URA + 2% glucose (v/v) overnight. 200 µL of culture liquid was collected to extract a genome, and with the genome as the template, verification was performed using primers 8 and 7 to obtain the correct transformants.

On the basis of the above, with AT02 as the starting strain, the recombinant strains AT02 with MAL12, MAL22, MAL32, IMA1, IMA2, IMA3, and IMA4 knocked out were obtained by repeating the steps of yeast transformation and verification (primers 15 and 16 and primers 21 and 22) using the recombinant plasmid MAL/IMA-gRNA and the repair fragments IMA-fragment and MAL-fragment. With AT02 as the starting strain, the recombinant strain AT03 with IMA5 knocked out was obtained by repeating the steps of yeast transformation and verification (primers 26 and 29) using the recombinant plasmid IMA5-gRNA and the repair fragment IMA5-fragment.

### Example 7 Construction of a recombinant strain with a sucrose synthetic pathway gene inserted

Insert fragment preparation: With a yeast genome as the template, PCR amplification was performed using primers 30 and 31, primers 32 and 33, primers 34 and 35, and primers 36 and 37, respectively, to obtain four gene fragments: an upstream homologous arm 106a-up, a downstream homologous arm 106a-down, a promoter TEF1p, and a terminator PGK1t. With a synthesized gene SUF 1 (SEQ ID NO. 7) optimized by a codon as the template, PCR amplification was performed using primers 38 and 39 to obtain a sucrose transporter fragment SUF 1. With these five gene fragments as the template, PCR amplification was performed using primers 30 and 33 to obtain an insert fragment SEQ ID NO. 8. With a yeast genome as the template, PCR amplification was performed using primers 42 and 43, primers 44 and 45, primers 46 and 47, primers 48 and 49, primers 50 and 51, and primers 56 and 57, respectively, to obtain an upstream homologous arm X2-up, a downstream homologous arm X2-down, a terminator ADH1t, a promoter CCW12p, a terminator FBA1t, and a promoter TDH3p. With sucrose phosphate phosphatase SPP (SEQ ID NO. 9) and sucrose phosphate synthase SPS (SEQ ID NO. 10) optimized by codons as the template, PCR amplification was performed using primers 52 and 53 and primers 54 and 55 to obtain genes SPP and SPS. With these eight gene fragments as the template, PCR amplification was performed using primers 42 and 45 to obtain an insert fragment SEQ ID NO. 11. With a yeast genome as the template, PCR amplification was performed using primers 60 and 61, primers 62 and 63, primers 64 and 65, primers 68 and 69, primers 70 and 71, and primers 72 and 73, respectively, to obtain an upstream homologous arm X3-up, a downstream homologous arm X3-down, a terminator PYK1t, a promoter TEF1p, a promoter ENO2p, and UDP-glucose pyrophosphorylase UGP1 (SEQ ID NO. 12) and a terminator thereof. With a mutant (SEQ ID NO. 13) of ADP-glucose pyrophosphorylase GlgC from the constructed *Escherichia coli* as the template, amplification was performed using primers 66 and 67 to obtain GlgC-TM (a GlgC mutant). With these seven gene fragments as the template, PCR amplification was performed using primers 60 and 62 to obtain an insert fragment SEQ ID NO. 14. With SEQ ID NO. 14 as the template, an insert fragment SEQ ID NO. 15 was obtained using primers 60 and 74. With SEQ ID NO. 11 as the template, an SPS-SPP expression cassette was obtained using primers 75 and 76, and with a yeast genome as the template, an sUGP1 fragment was obtained using primers 72 and 74. With the SPS-SPP expression cassette, the sUGP1 fragment, and the downstream homologous arm X3-down as the template, PCR amplification was performed using primers 72 and 62 to obtain an insert fragment SEQ ID NO. 16.

Gene insertion and verification: The recombinant yeast AT03 was transformed using a recombinant plasmid containing gRNA with 106a site and SEQ ID NO. 7, and the experimental steps were the same as above. Verification was performed using primers 40 and 41 to obtain a recombinant yeast strain AT04 containing a copy of sucrose transporter SUF1. With AT04 as the starting strain, transformation was performed using a plasmid containing gRNA with X2 site and the insert fragment SEQ ID NO. 11, and verification was performed using primers 58 and 47 and primers 50 and 59 to obtain a recombinant yeast AT05 containing a copy of SPP and a copy of SPS. With AT05 as the starting strain, transformation was performed using a plasmid containing gRNA with X3 site and the insert fragment SEQ ID NO. 14, and verification was performed using primers 78 and 65 and primers 72 and 79 to obtain a recombinant yeast AT06 containing a copy of UGP1 and a copy of GlgC-TM. With AT05 as the starting strain, transformation was performed using a plasmid containing gRNA with X3 site, the insert fragment SEQ ID NO. 15, and the insert fragment SEQ ID NO. 16, and verification was performed using primers 78 and 65, primers 72 and 49, and primers 50 and 79 to obtain a recombinant yeast AT07 containing a copy of UGP1, a copy of GlgC-TM, two copies of SPP, and two copies of SPS.

### Example 8 Method for producing sucrose by manes of transformation with a recombinant strain

### (1) Growth test with sucrose as a carbon source

AT03 was activated in YPD overnight, then collected, washed with sterile water twice, resuspended in sterile water for starvation treatment for 24 hours, and centrifuged to collect the yeast. The cells were resuspended with 1 mL of sterile water and inoculated in a liquid medium of YP + 2% sucrose, and after 24 hours, samples were collected to measure OD₆₀₀.

### (2) Fermentation to produce sucrose

AT04, AT05, AT06, and AT07 were selected and then activated in YPD overnight, and the activated strains were inoculated in 20 mL of YPE (2% ethanol), with starting OD₆₀₀ = 0.1, at 30 °C and 200 rpm for 96 hours. Samples were collected to measure OD₆₀₀ and subjected to sucrose content analysis.

### (3) Sucrose sample preparation

A sample was centrifuged at 13000 rpm for 10 minutes, and the supernatant was transferred to a new EP tube for extracellular sucrose content analysis. The cell precipitates were washed with water twice, resuspended with 80% ethanol, incubated at 60 °C for 4 hours, centrifuged to collect the supernatant, then blown dry with N₂ and dissolved with water for intracellular sucrose content measurement.

### (4) Sucrose content detection by LC-MS

The sample was filtered using a 0.45 µm filter tip and detected by LC-MS (Agilent 1290-6470). The chromatographic column was Agilent HILIC-OH5 (2.7 µm, 2.1 × 100 mm), the mobile phase A was water containing 5 mM ammonium formate and 0.1% formic acid, the mobile phase B was 80% acetonitrile containing 5 mM ammonium formate and 0.1% formic acid, the flow rate was 0.3 mL/min, the column temperature was 30 °C, and the sample volume was 2 µL.

### (5) Measurement result

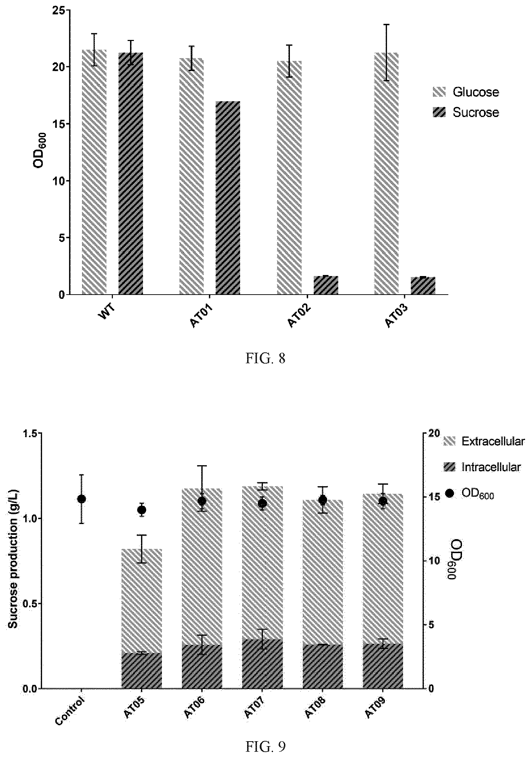

The recombinant strain AT01 constructed by knocking out the sucrose encoding gene SUC2 could continue to grow with sucrose as a carbon source (shown in FIG. 8). The applicant found that since maltase and isomaltase could also catalyze the degradation of sucrose, the recombinant strain AT02 was obtained by knocking out the maltase encoding genes MAL12, MAL22, and MAL32 and the isomaltase encoding genes IMA1, IMA2, IMA3, and IMA4, and the sucrose metabolism-deficient recombinant strain AT03 was obtained by further knocking out the isomaltase IMA5. The recombinant strain AT03 was constructed.

When glucose was used as the carbon source, there was no difference in growth between the wild-type strain and the recombinant strain, indicating that the knockout of these genes did not affect the health of the cells. When sucrose was used as the carbon source, the knockout of SUC2 reduced the sucrose metabolism capability of the strain, and when the maltase encoding genes MAL12, MAL22, and MAL32 and the isomaltase encoding genes IMA1, IMA2, IMA3, IMA4, and IMA5 were knocked out, the strain could not continue to consume sucrose for growth and thus could be used for chassis cells synthesized by means of biosynthesis with sucrose.

As can be seen in FIG. 9, no sucrose was detected in the wild-type strain WT intracellularly or extracellularly. After the heterologous expression of two sucrose synthesis pathway enzymes SPS and SPP, the recombinant strain AT05 significantly synthesized sucrose with an extracellular yield of 0.65 g/L and an intracellular yield of 0.20 g/L. When the precursors ADP-Glc and UDP-Glc were increased by overexpressing two enzymes GlgC and UGP1, respectively, the sucrose yield was significantly increased, the extracellular sucrose content was 0.88 g/L, the intracellular sucrose content was 0.13 g/L, and the total sucrose content was 1.01 g/L. When a copy of SPS and a copy of SPP were added on the basis of the above, the sucrose yield was not increased. The sequences of the primers 1 to 79 used to produce sucrose in Examples 6 to 8 of the present disclosure are shown in Table 9.

**Table 9 Sequences of the primers 1 to 79 used to produce sucrose in Examples 6 to 8 of the present disclosure**

| | | |
|---|---|---|
| 1 | 6006 | GTTTTAGAGCTAGAAATAGCAAGTTAAAATAAGGCTAGTC |
| 2 | SUC2-gRNA 1 | CCGCAGTGAAAGATAAATGATCGCATTACTAATGTTCAATATG TTTTAGAGCTAGAAATAGC |
| 3 | SUC2-gRNA2 | CCGCAGTGAAAGATAAATGATCTACATTAGCTATTCTCTTGAG TTTTAGAGCTAGAAATAGC |
| 4 | HoSUC2-up-F | GCCTAAGGGCTCTATAGTAAAC |
| 5 | HoSUC2-up-R | GACAATAAGTTTTATAACCTCATA TACGTTAGTGAAAAGAAAAG |
| 6 | HoSUC2-down-F | CTTTTCTTTTCACTAACGTATATGAGGTTATAAAACTTATTGTC |
| 7 | HoSUC2-down-R | GTAGTGTAAGGCAACTACATTAC |
| 8 | VSUC2-F | TCGAGGAATGCTTAAACGAC |
| 9 | MAL32-gRNA | CCGCAGTGAAAGATAAATGATCTATAAGGAACTTATGATTTAG TTTTAGAGCTAGAAATAGC |
| 10 | IMA-gRNA | CCGCAGTGAAAGATAAATGATCTTCTTCTGGAGACCTCCTAA GTTTTAGAGCTAGAAATAGC |
| 11 | HomoIMA-com-R | TTTTCGTAGTTGGCAATATCGTAACCCATATCATCTTGTGGCG AGTCG |
| 12 | HomoIMA-com-r | CAATGGTCTCGTGAGGAGCCAAATGCTGGTTTTCTGGTCCT A |
| 13 | HomoIMA-F | CGACTCGCCACAAGATGATATGGGTTACGATATTGCCAACTAC GAAAAGGTCTGGCCAACATGCTAGAACACCTATG |
| 14 | HomoIMA-R | TAGGACCAGAAAAACCAGCATTTGGCTCCTCACGAGACCATT GCATAGGTGTTCTAGCATGTTGGCCAGACC |
| 15 | IMA-verify-F | ATGACTATTTCTTCTGCACATCC |
| 16 | IMA-Verify-R | ATGGCTTCAATGTTCTGGAAG |
| 17 | HomoMAL-com-F | CCTCCCAAGAGAAGGTGCTTCTTTATCTTTTATTCTTGGAAA |
| 18 | HomoMAL-F | TTTTAAAGACTCCAATAACGATGGCTGGGGTGATTTAAAAGG TATCACTTCCAAGTTGCAGCGAAGAAATTGAATTCAG |
| 19 | HomoMAL-R | TTTCCAAGAATAAAAGATAAAGAAGCACCTTCTCTTGGGAGG CTGAATTCAATTTCTTCGCTGCAACTTGGAAGTG |
| 20 | HomoMAL-com-R | ATACCTTTTAAATCACCCCAGCCATCGTTATTGGAGTCTTTAA AA |
| 21 | MAL-Verify-F | ATGACTATTTCTGATCATCCAG |
| 22 | MAL-Verify-R | ATGGTTTCAAAACTCTGGAG |
| 23 | IMA5-gRNA 1 | CCGCAGTGAAAGATAAATGATCATGAATGAATCATTTAGAGA GTTTTAGAGCTAGAAATAGC |
| 24 | IMA5-gRNA | CCGCAGTGAAAGATAAATGATCTGGCCAACCTGATTTCAATT GTTTTAGAGCTAGAAATAGC |
| 25 | HomoIMA5-down-F | GTGCTCAATATTGAAAAGAAAAACCGAGCACATTTAACATAA ACG |
| 26 | HomoIMA5-down-R | GTTAGGGTGAGAATAGTCGAATG |
| 27 | HomoIMAS-up-F | AGTTGCGTGAAAGCGTAAATG |
| 28 | HomoIMAS-up-R | CGTTTATGTTAAATGTGCTCGGTTTTCTTTTCAATATTGAGCA C |
| 29 | IMA5-Verify-F | GATGCTCTAGGCTGCTTTCTC |
| 30 | 106a-up-F | GAGGGTCTGTCCAGCGAATAAG |
| 31 | tef106a-up-R | GGAGTAGAAACATTTTGAAGCTATCACAACCGACGATCCGGG GTC |
| 32 | pgk106a-down-f | GAAAATTCTGCGTTCGTTAGCTAATTTTTCCGGCAGAAAG |
| 33 | 106a-down-R | GAAGGAAAGGAAATCACTTGG |
| 34 | 106a-TEF-F | CCGGATCGTCGGTTGTGATAGCTTCAAAATGTTTCTACTCC |
| 35 | sufTEF-R | GATTCATTAGTAGATGGATTATCCATTTTGTAATTAAAACTTAG |
| | | ATTAG |
| 36 | sufPGK-F | CAATTGCTGGTGGTTTTCATTAAATTGAATTGAATTGAAATCG ATAGATC |
| 37 | 106a-PGK-R | CTTTCTGCCGGAAAAATTAGCTAACGAACGCAGAATTTTC |
| 38 | SUF 1-F | CTAATCTAAGTTTTAATTACAAAATGGATAATCCATCTACTAAT GAATC |
| 39 | SUF1-R | GATCTATCGATTTCAATTCAATTCAATTTAATGAAAACCACCA GCAATTG |
| 40 | 106a-verify-F | CCACATATTTTCCCTTTCTCTC |
| 41 | 106a-verify-R | GGAAGACACTAAAGGTACCTAGC |
| 42 | X-2 up fw | CGTCTATGAGGAGACTGTTAGTTG |
| 43 | X-2 up rv | GACCACTTCGAGAGCAAGTTG |
| 44 | X-2 down fw | CCTGCATAATCGGCCTCAC |
| 45 | X-2 down rv | CTCGCCAAGGCATTACCATC |
| 46 | x2ADH1t-F | CAACTTGCTCTCGAAGTGGTCGCGAATTTCTTATGATTTATG |
| 47 | ADH1t-R | GCATATCTACAATTGGGTGAAATG |
| 48 | CCW12-F | TTGAAATGGCAGTATTGATAATGATAAACTCGAAACCAGGGC AAAGCAAAATAAAAG |
| 49 | CCW12-R | TATTGATATAGTGTTTAAGCGAATGAC |
| 50 | FBA1t-F | GTTAATTCAAATTAATTGATATAG |
| 51 | FBA1t-R | GTGAGGCCGATTATGCAGGAGTAAGCTACTATGAAAGACTTT AC |
| 52 | SPP-F | AATCTTCTGTCATTCGCTTAAACACTATATCAATAATGAGACA ATTGTTGTTGATTTC |
| 53 | SPP-R | ATTAAAAAACTATATCAATTAATTTGAATTAACTTAAGACAAG AAATCAAAATGAGC |
| 54 | SPS-F | CGCTCCCCATTTCACCCAATTGTAGATATGCTTAAACTGGGTC TAACAATTCG |
| 55 | SPS-R | CTTAGTTTCGAATAAACACACATAAACAAACAAAATGTCTTA CTCCTCTAAGTAC |
| 56 | TDH3-F | TTTGTTTGTTTATGTGTGTTTATTCG |
| 57 | TDH3-R | TCGAGTTTATCATTATCAATACTG |
| 58 | X-2 det fw | TGCGACAGAAGAAAGGGAAG |
| 59 | X-2 det rv | GAGAACGAGAGGACCCAACAT |
| 60 | X-3 up fw | CGAGATCTTTGTGTTCGGTTACC |
| 61 | pykX3-UP-R | GAACTAACTTGGAAGGTTATACATGGGTACATAAATGCGTCTC GTATGTCGGCTCTCGC |
| 62 | X-3 down rv | GAGGTGGTTATTGATCACCGGA |
| 63 | ugpX3-down-F | GATCCTTTTCCTATTTTTCTCCTCTGTGTCCGCGTTTCTAAGGC |
| 64 | PYK1t-F | GCATTTATGTACCCATGTATAACC |
| 65 | PYK1-R | AAAAAGAATCATGATTGAATGAAG |
| 66 | glgCTM-F | CAAAAAAATAATATCTTCATTCAATCATGATTCTTTTTTTATCG CTCCTGTTTATGCCC |
| 67 | glgCTM-R | GAAAGCATAGCAATCTAATCTAAGTTTTAATTACAAAATGGTT AGTTTAGAGAAGAACG |
| 68 | TEF1p-F | TTTGTAATTAAAACTTAGATTAGATTG |
| 69 | TEF1p-R | ATAGCTTCAAAATGTTTCTACTC |
| 70 | tefENO2p-F | GAAGAGTAAAAAAGGAGTAGAAACATTTTGAAGCTATACGC GGCGTTATGTCACTAAC |
| 71 | ENO2p-R | TATTATTGTATGTTATAGTATTAGTTG |
| 72 | UGP1-F | ATAACACCAAGCAACTAATACTATAACATACAATAATAATGTC CACTAAGAAGCACACC |
| 73 | UGP1-R | GCCTTAGAAACGCGGACACAGAGGAGAAAAATAGGAAAAG GATC |
| 74 | adhUGP1-R | TTAATAATAAAAATCATAAATCATAAGAAATTCGCGAGGAGAA AAATAGGAAAAGGATC |
| 75 | ADH1-F | GCGAATTTCTTATGATTTATGATTT |
| 76 | FBA1t-R | AGTAAGCTACTATGAAAGACTTTAC |
| 77 | fbaX3-down-F | TCTTCGAGTTCTTTGTAAAGTCTTTCATAGTAGCTTACTTGTG TCCGCGTTTCTAAGGC |
| 78 | X-3 Verify fw | TGACGAATCGTTAGGCACAG |
| 79 | X-3 Verify rv | CCGTGCAATACCAAAATCG |

### Example 9 Preparation of a recombinant strain with a glucosamine utilization deficiency, a capability to secrete glucosamine and an improved yield

### (1) Glucosamine synthesis pathway construction

Insert fragment preparation: With a yeast genome as the template, PCR amplification was performed using primers b1 and b2, primers b3 and b4, primers b5 and b6, primers b7 and b8, primers b9 and b10, and primers b15 and b16, respectively, to obtain six gene fragments: an upstream homologous arm X2-up, a downstream homologous arm X2-down, a terminator ADH1t, a promoter CCW1p, a terminator FBA1t, and a promoter TDH3p. With synthesized genes GlmD (SEQ ID NO. 17) and GlmP (SEQ ID NO. 18) optimized by codons as the template, PCR amplification was performed using primers b11 and b12 to obtain a glucosamine-6-phosphate deaminase fragment GlmD from *Bacillus subtilis,* and PCR amplification was performed using primers b13 and b14 to obtain a glucosamine-6-phosphate phosphatase fragment GlmP from *Bacteroides thetaiotaomicron.* With these eight gene fragments as the template, PCR amplification was performed using primers b1 and b4 to obtain an insert fragment SEQ ID NO. 19. The PCR procedure was the same as above. With the CT01 genome as the template, PCR amplification was performed using primers b19 and b20, primers b21 and b22, and primers b23 and b24, respectively, to obtain three fragments: an upstream homologous arm X3-up, a GlmD-GlmP expression cassette, and a downstream homologous arm X3-down. With these three gene fragments as the template, PCR amplification was performed using primers b19 and b24 to obtain an insert fragment SEQ ID NO. 20. The PCR procedure was the same as above. With the CT01 genome as the template, PCR amplification was performed using primers b27 and b28 and primers b29 and b30, respectively, to obtain two fragments: an upstream homologous arm XII-5-up and a downstream homologous arm XII-5-down. With these two gene fragments and the GlmD-GlmP expression cassette as the template, PCR amplification was performed using primers b27 and b30 to obtain an insert fragment SEQ ID NO. 21. The PCR procedure was the same as above. With the CT01 genome as the template, PCR amplification was performed using primers b33 and b34, primers b35 and b36, and primers b37 and b38, respectively, to obtain three fragments: an upstream homologous arm XI-1-up, a downstream homologous arm XI-1-down, and a GlmP expression cassette. With these three gene fragments as the template, PCR amplification was performed using primers b33 and b36 to obtain an insert fragment SEQ ID NO. 22.

Gene insertion and verification: The yeast LY031 was transformed using a recombinant plasmid containing gRNA with X2 site and SEQ ID NO. 19, and the experimental steps were the same as above. Verification was performed using primers b17 and b8 and primers b7 and b 18 to obtain a recombinant yeast CT01 containing a copy of GlmD and a copy of GlmP. The yeast CT01 was transformed using a recombinant plasmid containing gRNA with X3 site and XII-5 site, SEQ ID NO. 20, and SEQ ID NO. 21, and the experimental steps were the same as above. Verification was performed using primers b25 and b8, primers b7 and b26, primers b31 and b8, and primers b7 and b 32 to obtain a recombinant yeast CT02 containing three copies of GlmD and three copies of GlmP. The yeast CT02 was transformed using a recombinant plasmid containing gRNA with XI-1 site and SEQ ID NO. 22, and the experimental steps were the same as above. Verification was performed using primers b39 and b40 to obtain a recombinant yeast strain CT03 containing three copies of GlmD and four copies of GlmP.

### (2) REG1 knockout

REG1 knockout was performed in the same way as the knockout of the glucose strains in Examples 1 and 2 above to obtain a recombinant yeast CT04 with RGE1 knocked out.

### Example 10 Method for producing glucosamine by means of transformation with a recombinant strain

### (1) Glucosamine fermentation and detection

LY031, CT01, CT02, CT03, and CT04 were selected and then activated in YPE overnight, and the activated strains were inoculated in 20 mL of YPE (2% ethanol), with starting OD₆₀₀ = 0.1, at 30 °C and 200 rpm for 96 hours. Samples were collected to measure OD₆₀₀ and subjected to glucosamine content analysis.

The sample was filtered using a 0.45 µm filter tip and detected by LC-MS (Agilent 1290-6470). The chromatographic column was Agilent HILIC-OH5 (2.7 µm, 2.1 × 100 mm), the mobile phase A was water containing 5 mM ammonium formate and 0.1% formic acid, the mobile phase B was 80% acetonitrile containing 5 mM ammonium formate and 0.1% formic acid, the flow rate was 0.3 mL/min, the column temperature was 30 °C, and the sample volume was 2 µL.

### (2) Experimental result

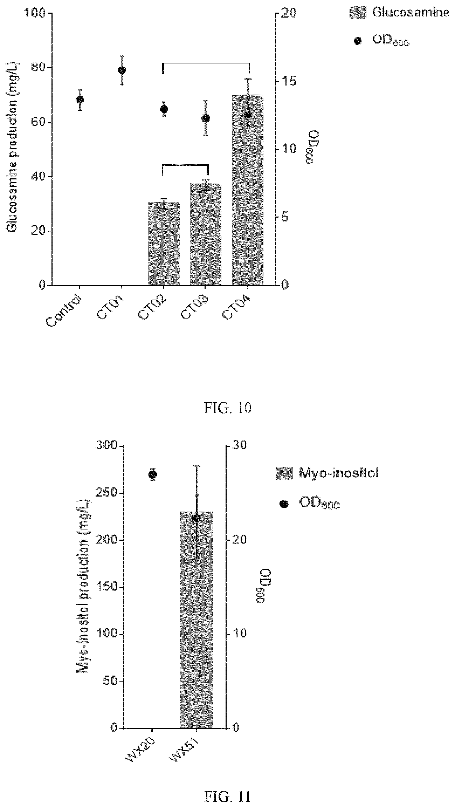

As shown in FIG. 10, the synthesis of glucosamine was not detected in the recombinant yeast CT01, while CT02 could produce glucosamine with a yield of 30.09 mg/L. To further improve the glucosamine yield, an additional copy of GlmP was integrated into the genome of CT02, and the experimental results showed that the glucosamine yield of CT03 was improved by 23.09%. On the basis of the above, the REG1 knockout strain CT04 could further improve the glucosamine yield to 69.66 mg/L, 1.31 times higher than CT02.

The sequences of the primers b1 to b40 used to produce glucosamine in Examples 9 to 10 of the present disclosure are shown in Table 10.

**Table 10 Sequences of the primers b1 to b40 used to produce glucosamine in Examples 9 to 10 of the present disclosure**

| | Primers | Sequences |
|---|---|---|
| b1 | X-2 up fw | CGTCTATGAGGAGACTGTTAGTTG |
| b2 | X-2 up rv | GACCACTTCGAGAGCAAGTTG |
| b3 | X-2 down fw | CCTGCATAATCGGCCTCAC |
| b4 | X-2 down rv | CTCGCCAAGGCATTACCATC |
| b5 | x2ADH1t-F | CAACTTGCTCTCGAAGTGGTCGCGAATTTCTTATGATTTAT G |
| b6 | ADH1t-R | GCATATCTACAATTGGGTGAAATG |
| b7 | CCW12-F | TTGAAATGGCAGTATTGATAATGATAAACTCGAAACCAGG |
| | | GCAAAGCAAAATAAAAG |
| b8 | CCW12-R | TATTGATATAGTGTTTAAGCGAATGAC |
| b9 | FBA1t-F | GTTAATTCAAATTAATTGATATAG |
| b10 | x2FBA1t-R | GTGAGGCCGATTATGCAGGAGTAAGCTACTATGAAAGACT TTAC |
| b11 | GlmD-F | CAAATCGCTCCCCATTTCACCCAATTGTAGATATGCTTATG GTCTCAAAGAAGCAGC |
| b12 | GlmD-R | AACTTAGTTTCGAATAAACACACATAAACAAACAAAATGA AGGTTATGGAATGTCAAAC |
| b13 | GlmP-F | AATCTTCTGTCATTCGCTTAAACACTATATCAATAATGATTA AGGTTTTGTTGTTGG |
| b14 | GlmP-R | ACTCATTAAAAAACTATATCAATTAATTTGAATTAACTTAAA TAACACCGAAATGCTTC |
| b15 | TDH3-F | TTTGTTTGTTTATGTGTGTTTATTCG |
| b16 | TDH3-R | TCGAGTTTATCATTATCAATACTG |
| b17 | X-2 det fw | TGCGACAGAAGAAAGGGAAG |
| b18 | X-2 det rv | GAGAACGAGAGGACCCAACAT |
| b19 | X-3 up fw | CGAGATCTTTGTGTTCGGTTACC |
| b20 | adhX3-UP-R | TATTTAATAATAAAAATCATAAATCATAAGAAATTCGCGTCT CGTATGTCGGCTCTCGC |
| b21 | ADH1t-F | GCGAATTTCTTATGATTTATGATT |
| b22 | FBA1t-R | AGTAAGCTACTATGAAAGACTTTAC |
| b23 | fb a 1 X3 -down-F | TCTTCGAGTTCTTTGTAAAGTCTTTCATAGTAGCTTACTTGT GTCCGCGTTTCTAAGGC |
| b24 | X-3 down rv | GAGGTGGTTATTGATCACCGGA |
| b25 | X-3 Verification fw | TGACGAATCGTTAGGCACAG |
| b26 | X-3 Verification rv | CCGTGCAATACCAAAATCG |
| b27 | XII-5 up F | GTAGTGATCATTGGCTTAACG |
| b28 | adh1XII5-UP-R | ATTTAATAATAAAAATCATAAATCATAAGAAATTCGCGTGA CAATAAATTCAAACCGGT |
| b29 | fba1XII5-down-F | TCTTCGAGTTCTTTGTAAAGTCTTTCATAGTAGCTTACTCA ACTCAGAAGTTTGACAGC |
| b30 | XII-5 down R | CTCTTTTGCCTTTCAAAAAAG |
| b31 | XII-5 det fw | CCACCGAAGTTGATTTGCTT |
| b32 | XII-5 det rv | GTGGGAGTAAGGGATCCTGT |
| b33 | XI-1 up F | ATTTGTGTGAAGGAATAGTGACG |
| b34 | XI-1 up R | CAATGGGCTTGGTATTCCG |
| b35 | XI-1 down F | TTTCTTGGCATTGGCAAATC |
| b36 | XI-1 down R | AAGAGCCGAGTCCCCATCAG |
| b37 | XI-1-GlmP-F | CGGAATACCAAGCCCATTGAACCAGGGCAAAGCAAAATA AAAG |
| b38 | XI-1-GlmP-R | GATTTGCCAATGCCAAGAAAAGTAAGCTACTATGAAAGAC TTTAC |
| b39 | XI-1 det fw | CTTAATGGGTAGTGCTTGACACG |
| b40 | XI-1 det rv | GAAGACCCATGGTTCCAAGGA |
| b41 | reg1-up-F | GTGGATATTGAAGGAAGGAATCAG |
| b42 | reg1-up-R | CTTCATGTTGACTTCAAAATTCTTTCTTTTTTGGATTTTTCT TATCTCGTCTTCG |
| b43 | reg1-dn-F | GAAGACGAGATAAGAAAAATCCAAAAAAGAAAGAATTTT GAAGTCAACATGAAG |
| b44 | reg1-dn-R | GGTGTTTCGCTTATTGACATTG |
| b45 | reg1-VF | GCCTCTTTTCTGACATAATATTGG |
| b46 | reg1-VR | CCCTCAGGAACAACAATTTTAGG |
| b47 | reg1-gRNA | CGCAGTGAAAGATAAATGATCAAAATCCTACTAATTTTACA GTTTTAGAGCTAGAAATAGCAAGT |

### Example 11 Preparation of a recombinant strain with an inositol utilization deficiency and a capability to secrete inositol

### (1) Knockout of genes PFK1 and PFK2

Knockout plasmid construction: With pROS10 as the template, the plasmid skeleton was amplified using a primer c1, the 2 µm fragment containing PFK1-gRNA and PFK2-gRNA was amplified using primers c2 and c3, and the PCR procedure was performed according to the instructions of the 2× Taq enzyme. The plasmid skeleton and the 2 µm fragment containing PFK1-gRNA and PFK2-gRNA were added to a Gibson reaction system in an equal molar ratio and incubated at 50 °C for 1 hour, the system was transformed to *Escherichia coli* DH5a competent cells, and the cells were revived for 40 minutes and coated on an Amp-resistant LB plate. After 12 hours of culture at 37 °C, sequencing and plasmid extraction were performed on a single colony to obtain a PFK1/PFK2 double-site knockout plasmid.

Homologous fragment preparation: Primers c4 to c7 were synthesized, and the dry powder of each primer was dissolved to a concentration of 100 µM with ddH₂O. Primers c4 and c5 and primers c6 and c7 were mixed at 1:1, respectively, heated at 95 °C for 5 minutes to fuse the mixed primers, and then cooled at room temperature to obtain the repair fragments PFK1-fragment and PFK2-fragment.

Gene knockout and validation: The competent cells of the strain Lab001 were prepared, and the Lab001 was transformed using 1 µg of PFK1/PFK2 double-site knockout plasmid obtained in the above step, 2 µL of the repair fragment PFK1-fragment, and 2 µL of the repair fragment PFK2-fragment, coated on an SE-URA plate, and cultured at 30 °C. After 3 days of culture, a recombinant strain grew. A single colony was placed in an SE-URA liquid medium, and 36 hours later, a genome was extracted with 200 µL of yeast liquid. With the genome as the template, verification was performed using primers c8 and c9 and primers c10 and c11 to obtain a correct recombinant strain WX20.

### (2) Inositol synthesis pathway construction

Insert fragment preparation: With *Saccharomyces cerevisiae* IMX581 as the template, PCR amplification was performed using primers c12 and c13, primers c14 and c15, primers c16 and c17, primers c18 and c19, primers c20 and c21, primers c22 and c23, and primers c24 and c25, respectively, to obtain seven fragments: an upstream fragment XI-2 up, promotors TDH3p and TEF1p, terminator CYC1t and ADH2t, a gene GDH2, and a downstream homologous arm XI-2 down. With a codon-optimized synthesized gene *suhB* (SEQ ID NO. 23) from *Escherichia coli* as the template, the *suhB* fragment was amplified using primers c26 and c27. With these eight gene fragments as the template, PCR amplification was performed using primers c12 and c17 and primers c16 and c25, respectively, to obtain insert fragments IP-1 (SEQ ID NO. 24) and IP-2 (SEQ ID NO. 25).

PCR amplification was performed using primers c28 and c29, primers c30 and c31, primers c32 and c33, primers c34 and c35, primers c36 and c37, primers c38 and c39, primers c40 and c41, and primers c42 and c43, respectively, to obtain eight fragments: an upstream fragment XII-3 up, promotors PGKIp and CCW12p, terminator PYK1t and FBA1t, genes INO1 (SEQ ID NO. 26) and ITR1 (SEQ ID NO. 27), and a downstream homologous arm XII-3 down. With these eight gene fragments as the template, PCR amplification was performed using primers c28 and c33 and primers c32 and c43, respectively, to obtain fusion fragments IP-3 (SEQ ID NO. 28) and IP-4 (SEQ ID NO. 29).

Gene insert and validation: The competent cells of the strain WX20 were prepared, and the WX20 was transformed using 500 ng of recombinant plasmid containing gRNA with XI-2 site and XII-3 site, 500 ng of fusion fragment IP-1, 500 ng of fusion fragment IP-2, 500 ng of fusion fragment IP-3, and 500 ng of fusion fragment IP-4, coated on an SE-URA plate, and cultured at 30 °C. After 3 days of culture, a recombinant strain grew. A single colony was cultured for 36 hours, and a genome was extracted with 200 µL of yeast liquid. With the genome as the template, verification was performed using primers c44 and c45 and primers c46 and c47 to obtain a correct recombinant strain WX51.

### Example 12 Method for producing inositol by means of transformation with a recombinant strain

### (1) Fermentation to produce inositol

WX20 and WX51 monoclonal strains were activated in YPE, and then the activated seed broth was inoculated in 20 mL of YPDE (2% ethanol + 0.5% ethanol), with starting OD₆₀₀ = 0.1, at 30 °C and 200 rpm for 72 hours. Samples were collected to measure final OD₆₀₀, and other samples were collected to analyze inositol content.

### (2) Inositol content measurement

1.2 mL of sample fermentation liquid was centrifuged at 3000 g for 5 minutes, filtered through a 0.22 µm filter tip, and detected by HPLC (Agilent 1260b). The chromatographic column was Aminex HPX-87H (300 mm × 7.8 mm), the mobile phase was 5 mM sulfuric acid, the flow rate was 0.6 mL/min, the column temperature was 50 °C, and the sample volume was 5 µL.

As shown in FIG. 11, the synthesis of inositol was not detected in the recombinant yeast WX20, while the inositol yield of WX51 was 228.71 mg/L.

The sequences of the primers c1 to c47 used to produce inositol in Examples 11 to 12 of the present disclosure are shown in Table 11.

**Table 11 Sequences of the primers c1 to c47 used to produce inositol in Examples 11 to 12 of the present disclosure**

| | | |
|---|---|---|
| c1 | 6005 | GATCATTTATCTTTCACTGCGGAGAAG |
| c2 | PFK1-gRNA1 | TGCGCATGTTTCGGCGTTCGAAACTTCTCCGCAGTGAAAGATA AATGATCGTGGCTGGAATCAAACACATGTTTTAGAGCTAGAAA TAGCAAGTTAAAATAAG |
| c3 | PFK2-gRNA2 | TGCGCATGTTTCGGCGTTCGAAACTTCTCCGCAGTGAAAGATA AATGATCTAGCTGGTATCAAGACCATTGTTTTAGAGCTAGAAAT AGCAAGTTAAAATAAG |
| c4 | PFK1-repair-F | ACTTTAGCACTATTTGGGAAAGCTTTTATATAAAAAATCTGAAA CAAAATCATATCAAAGATGATTGCAATGAAAAGTTTAAGTTAA GCAAAAGGAGGTAAAAATGGCATGCACTTTAAT |
| c5 | PFK1-repair-R | ATTAAAGTGCATGCCATTTTTACCTCCTTTTGCTTAACTTAAACT TTTCATTGCAATCATCTTTGATATGATTTTGTTTCAGATTTTTTAT ATAAAAGCTTTCCCAAATAGTGCTAAAGT |
| c6 | PFK2-repair-F | TGAACAATAGAACTAGATTTAGAGACTAGTTTAGCATTGGCCA AGAACTAACCATACGCAAAGAAAATGACCTTTTATTACACTTTC TATTATTAATGTCAATTAATGTTAACCCATGTT |
| c7 | PFK2-repair-R | AACATGGGTTAACATTAATTGACATTAATAATAGAAAGTGTAAT AAAAGGTCATTTTCTTTGCGTATGGTTAGTTCTTGGCCAATGCT AAACTAGTCTCTAAATCTAGTTCTATTGTTCA |
| c8 | PFK1-VER-F | TCCGATTTGAGATCGACTTG |
| c9 | PFK 1-VER-R | TAGTTTCCATTTTTCCAGCG |
| c10 | PFK2-VER-F | TCGTTCCAAATGGCGTCCAC |
| c11 | PFK2-VER-R | TTCCTGAGAGTTATCAGACG |
| c12 | XI-2 up-F | TAACTCTTCGTATGAGGATTTTC |
| c13 | XI-2 up-R | TTAATTTGCGGCCGGTACCCTTCTATGGCACATTTTTCTGTTG |
| c14 | TDH3p-F | TAGAAACATTTTGAAGCTATTCGAGTTTATCATTATCAAT |
| c15 | TDH3p-R | CATTTTGTTTGTTTATGTGTG |
| c16 | TEF1p-F | ATTGATAATGATAAACTCGAATAGCTTCAAAATGTTTCTAC |
| c17 | TEF1p-F | CATTTTGTAATTAAAACTTAG |
| c18 | CYC1t-F | GATACCGTCGACCTCGAGTCAT |
| c19 | CYC1t-R | CAGAAAAATGTGCCATAGAAGGGTACCGGCCGCAAATTAA |
| c20 | ADH2t-F | AAGCGGAGGCAAGTGCTTGAGCGAATTTCTTATGATTTATGATT T |
| c21 | ADH2t-R | CAACGAGCTTTACTTGTGGGCATATCTACAATTGGGTGAA |
| c22 | GDH2-F | GAAAGCATAGCAATCTAATCTAAGTTTTAATTACAAAATGATGC TTTTTGATAACAAAAAT |
| c23 | GDH2-R | ATAAATCATAAGAAATTCGCTCAAGCACTTGCCTCCGCTT |
| c24 | XI-2 down-F | TTCACCCAATTGTAGATATGCCCACAAGTAAAGCTCGTTGAC |
| c25 | XI-2 down-R | ATGGTTGAAAAGGTTACAGAGG |
| c26 | suhB-F | TTAGTTTCGAATAAACACACATAAACAAACAAAATGATGCACC CTATGCTTAACATAG |
| c27 | suhB-R | ATAACTAATTACATGACTCGAGGTCGACGGTATCTTATCTCTTC AAAGCATCAGAC |
| c28 | XII-3-up-F | TGTGCCCCTTAAAATTCATATAC |
| c29 | XII-3-up-R | ATACATGGGTACATAAATGCGAATGAGCAGGTACCCCTTA |
| c30 | PGK1p-F | TATTTTGCTTTGCCCTGGTTACGCACAGATATTATAACATC |
| c31 | PGK1p-R | TTTGTTATATTTGTTGTAAAAAG |
| c32 | CCW12p-F | ATGTTATAATATCTGTGCGTAACCAGGGCAAAGCAAAATAAAA G |
| c33 | CCW12p-R | TATTGATATAGTGTTTAAGCGAATG |
| c34 | PYK1t-F | AAAAAGAATCATGATTGAATGAAGATA |
| c35 | PYK1t-R | TAAGGGGTACCTGCTCATTCGCATTTATGTACCCATGTATAAC |
| c36 | FBA1t-F | GTTAATTCAAATTAATTGATATAGTTT |
| c37 | FBA1t-R | CAAACCTAATTAGCTCTATGCAGTAAGCTACTATGAAAGACT |
| c38 | INO1-F | GTAATTATCTACTTTTTACAACAAATATAACAAAATGACAGAAG ATAATATTGCTCC |
| c39 | INO1-R | AAATAATATCTTCATTCAATCATGATTCTTTTTTTACAACAATCT CTCTTCGAAT |
| c40 | ITR1-F | TAATCTTCTGTCATTCGCTTAAACACTATATCAATAATGGGAATA CACATACCATATC |
| c41 | ITR1-R | TCATTAAAAAACTATATCAATTAATTTGAATTAACCTATATATCCT CTATAATCTCTTG |
| c42 | XII-3 down-F | AGTCTTTCATAGTAGCTTACTGCATAGAGCTAATTAGGTTTG |
| c43 | XII-3 | GAACTTACAAGCTGATTTTGGT |
| | down-R | |
| c44 | XI-2 VER-F | GTTTGTAGTTGGCGGTGGAG |
| c45 | XI-2 VER-R | GAGACAAGATGGGGCAAGAC |
| c46 | XII-3 VER-F | TGGGCAGCCTTGAGTAAATC |
| c47 | XII-3 VER-R | TGGCCAATTGTTCAGTCAAG |

The above are only specific embodiments of the present disclosure, not all embodiments. Any equivalent modifications of the technical solutions of the present disclosure made by those of ordinary skill in the art after reading the specification of the present disclosure are covered by the claims of the present disclosure.

## Claims

1. A construction method for a recombinant yeast strain for the production of glucose and derivatives thereof, comprising any one of the following (i-iii):
i, knocking out metabolic pathway-related enzymes of glucose and derivatives thereof in a yeast strain;
ii, enhancing or using an activity of synthetic pathway-related enzymes of glucose and derivatives thereof in the yeast strain; and
iii, enhancing or using a capability of glucose and derivatives thereof in the yeast strain to enter and exit the yeast;
preferably, the yeast strain comprises *Saccharomyces cerevisiae, Pichia pastoris,* and a *Yarrowia lipolytica,* more preferably, *Saccharomyces cerevisiae* and *Pichiapastoris.*

2. The construction method according to claim 1, wherein the metabolic pathway-related enzymes of glucose comprise glucokinase and related hexokinase isoenzymes; and metabolic pathway-related enzymes of sucrose in glucose derivatives comprise sucrase, maltase, and isomaltase;
the synthetic pathway-related enzymes of glucose comprise glucose phosphatase, and preferably, glucose phosphatase comprises glucose-1-phosphatase and glucose-6-phosphatase; synthetic pathway-related enzymes of glucosamine in the glucose derivatives comprise glucosamine-6-phosphate phosphatase and glucosamine-6-phosphate deaminase; synthetic pathway-related enzymes of sucrose in the glucose derivatives comprise sucrose phosphate phosphatase, sucrose phosphate synthase, UDP-glucose pyrophosphorylase, and ADP-glucose pyrophosphorylase; synthetic pathway-related enzymes of inositol in the glucose derivatives comprise inositol-3-phosphate synthase and inositol monophosphatase;
a protein that enhances a capability of sucrose in the glucose derivatives to enter and exit the yeast comprises a sucrose transporter; and a protein that enhances a capability of inositol in the glucose derivatives to enter and exit the yeast comprises an inositol transporter.

3. A recombinant yeast strain, comprising any one of the following (1-4):
1) a recombinant yeast strain A with a glucose utilization deficiency and a capability to secrete glucose, which is obtained by knocking out encoding genes of glucokinase and related hexokinase isoenzymes in *Saccharomyces cerevisiae*/*Pichia pastoris*;
2) a recombinant yeast strain B with a capability to secrete glucosamine, which is obtained by inserting several copies of glucosamine-6-phosphate phosphatase GlmP and glucosamine-6-phosphate deaminase GlmD into a recombinant yeast strain E with an improved glucose synthesis yield which is used as a starting strain, wherein the recombinant yeast strain E is obtained by optionally knocking out hexokinase isoenzyme genes in the recombinant yeast strain A described in 1) and overexpressing glucose phosphatase and HAD4 of *Escherichia coli* or overexpressing HAD4 of *Escherichia coli,* and preferably, three copies of GlmD and three copies of GlmP are inserted or three copies of GlmD and four copies of GlmP are inserted;
3) a recombinant yeast strain C with a sucrose utilization deficiency and a capability to secrete sucrose, which is obtained by knocking out encoding genes of sucrase, maltase, and isomaltase in a yeast, inserting a sucrose transporter SUF 1, and inserting one or more copies of sucrose phosphate phosphatase SPP and sucrose phosphate synthase SPS; and
4) a recombinant yeast strain D with an inositol utilization deficiency and a capability to secrete inositol, which is obtained by inserting endogenous inositol-3-phosphate synthase INO1 and exogenous inositol monophosphatase SuhB into a yeast and inserting an inositol transporter ITR1;
wherein, preferably, the yeast comprises *Saccharomyces cerevisiae, Pichia pastoris,* and a *Yarrowia lipolytica.*

4. The recombinant yeast strain according to claim 3, wherein a specific method for constructing the recombinant yeast strain A described in 1) is: when *Saccharomyces cerevisiae* is selected, knocking out a glucokinase gene *glkl* having a glucokinase activity and two hexokinase isoenzyme genes *hxkl* and *hxk2* in *Saccharomyces cerevisiae* to obtain a recombinant *Saccharomyces cerevisiae* strain A with a glucose utilization deficiency and a capability to secrete glucose; and
when *Pichia pastoris* is selected, knocking out a glucokinase gene *glkl* having a glucokinase activity and a hexokinase isoenzyme gene *hxkl* in *Pichiapastoris* to obtain a recombinant *Pichia pastoris* strain A with a glucose utilization deficiency and a capability to secrete glucose;
a specific method for constructing the recombinant yeast strain C described in 3) is: when the yeast is *Saccharomyces cerevisiae,* knocking out a sucrase active gene *suc2,* maltase active genes *mal12, mal22,* and *mal32,* and isomaltase active genes *ims1, ima2, ima3, ima4,* and *ima5* to obtain a recombinant *Saccharomyces cerevisiae* strain with a sucrose utilization deficiency, and overexpressing a sucrose transporter SUF1 from pea and sucrose phosphate phosphatase SPP and sucrose phosphate synthase SPS from polycystis to obtain a recombinant yeast strain C with a sucrose utilization deficiency and a capability to secrete sucrose.

5. The recombinant yeast strain according to claim 3, wherein, the recombinant yeast strain preferably is:
a) the recombinant yeast strain E with an improved glucose synthesis yield, which is obtained by optionally knocking out hexokinase isoenzyme genes in the recombinant yeast strain A and overexpressing glucose phosphatase and HAD4 of *Escherichia coli* or overexpressing HAD4 of *Escherichia coli*;
b) a recombinant yeast strain F with an improved glucosamine synthesis yield, which is obtained by knocking out a yeast endogenous gene *reg1* in the recombinant yeast strain B;
c) a recombinant yeast strain G with an improved sucrose synthesis yield, which is obtained by inserting a glucose pyrophosphorylase GlgC mutant and UGP1 into the recombinant yeast strain C and increasing precursor substances ADP-Glc and UDP-Glc, respectively;
d) a recombinant yeast strain H with an improved inositol synthesis yield, which is obtained by knocking out phosphofructokinase 1 and phosphofructokinase 2 in the recombinant yeast strain D and overexpressing glutamate transhydrogenase GDH1.

6. The recombinant yeast strain according to claim 3 or 5, wherein glucose phosphatase possesses glucose-1-phosphate and/or glucose-6-phosphate activities, and glucose phosphatase, glucose pyrophosphorylase GlgC/UGP1, glutamate transhydrogenase, glucosamine-6-phosphate phosphatase GlmP, glucosamine-6-phosphate deaminase GlmD, sucrose phosphate phosphatase SPP, sucrose phosphate synthase SPS, sucrose transporter, inositol-3-phosphate synthase, inositol monophosphatase, and inositol transporter are derived from heterologous enzymes or specific modified enzymes of the yeast itself or other eukaryotic and prokaryotic organisms.

7. The recombinant yeast strain according to claim 5, wherein a specific method for constructing the recombinant yeast strain E described in a) is: knocking out hexokinase genes *emi2* and *YLR446W* in a recombinant *Saccharomyces cerevisiae* A with a glucose utilization deficiency and a capability to secrete glucose and overexpressing a glucose phosphatase gene *agpP* from *Pantoea* and an HAD4 gene *yihx* from *Escherichia coli* at *YLR446W* and *emi2* sites, respectively, or knocking out hexokinase genes *emi2* and *YLR446W* in a recombinant *Saccharomyces cerevisiae* A with a glucose utilization deficiency and a capability to secrete glucose and overexpressing an HAD4 gene *yihx* from *Escherichia coli* at an *emi2* site, to obtain the recombinant yeast strain E with an improved glucose synthesis yield;
a specific method for constructing the recombinant yeast strain E described in a) is: when the recombinant yeast strain A is constructed by selecting *Pichia pastoris* as an original yeast, knocking out hexokinase isoenzyme genes *hxk2* and *hxk iso2* and overexpressing an HAD4 gene *yihx* from *Escherichia coli* at an *hxk iso2* site to obtain the recombinant yeast strain E with an improved glucose synthesis yield;
a specific method for constructing the recombinant yeast strain G described in c) is: overexpressing UDP-glucose pyrophosphorylase UGP1 derived from *Saccharomyces cerevisiae* and a GlgC mutant of ADP-glucose pyrophosphorylase derived from *Escherichia coli* to obtain the recombinant *Saccharomyces cerevisiae* strain G with a high sucrose yield.

8. The recombinant yeast strain according to claim 4, 6 or 7, wherein a nucleotide sequence of *agpP* is as shown in SEQ ID NO. 1, or a sequence having at least 70% homology therewith; an amino acid sequence of a protein formed after the expression of *agpP* is shown in SEQ ID NO. 32, or a sequence having at least 70% homology therewith;
a nucleotide sequence of *yihx* is as shown in SEQ ID NO. 2, or a sequence having at least 70% homology therewith; an amino acid sequence of a protein formed after the expression of *yihx* is shown in SEQ ID NO. 33, or a sequence having at least 70% homology therewith;
a nucleotide sequence of GlmD is as shown in SEQ ID NO. 17, or a sequence having at least 70% homology therewith; an amino acid sequence of a protein formed after the expression of GlmD is shown in SEQ ID NO. 34, or a sequence having at least 70% homology therewith;
a nucleotide sequence of GlmP is as shown in SEQ ID NO. 18, or a sequence having at least 70% homology therewith; an amino acid sequence of a protein formed after the expression of GlmP is shown in SEQ ID NO. 35, or a sequence having at least 70% homology therewith;
a nucleotide sequence of SLTF 1 is as shown in SEQ ID NO. 7, or a sequence having at least 70% homology therewith; an amino acid sequence of a protein formed after the expression of SUF1 is shown in SEQ ID NO. 36, or a sequence having at least 70% homology therewith;
a nucleotide sequence of SPP is as shown in SEQ ID NO. 9, or a sequence having at least 70% homology therewith; an amino acid sequence of a protein formed after the expression of SPP is shown in SEQ ID NO. 37, or a sequence having at least 70% homology therewith;
a nucleotide sequence of SPS is as shown in SEQ ID NO. 10, or a sequence having at least 70% homology therewith; an amino acid sequence of a protein formed after the expression of SPS is shown in SEQ ID NO. 38, or a sequence having at least 70% homology therewith;
a nucleotide sequence of UGP1 is as shown in SEQ ID NO. 12, or a sequence having at least 70% homology therewith; an amino acid sequence of a protein formed after the expression of UGP1 is shown in SEQ ID NO. 39, or a sequence having at least 70% homology therewith;
a nucleotide sequence of the GlgC mutant is as shown in SEQ ID NO. 13, or a sequence having at least 70% homology therewith; an amino acid sequence of a protein formed after the expression of the GlgC mutant is shown in SEQ ID NO. 40, or a sequence having at least 70% homology therewith;
a nucleotide sequence of INO1 is as shown in SEQ ID NO. 26, or a sequence having at least 70% homology therewith; an amino acid sequence of a protein formed after the expression of INO1 is shown in SEQ ID NO. 41, or a sequence having at least 70% homology therewith;
a nucleotide sequence of ITR1 is as shown in SEQ ID NO. 27, or a sequence having at least 70% homology therewith; an amino acid sequence of a protein formed after the expression of ITR1 is shown in SEQ ID NO. 42, or a sequence having at least 70% homology therewith;
a nucleotide sequence of a mutant of GDH1 is as shown in SEQ ID NO. 30, or a sequence having at least 70% homology therewith; an amino acid sequence of a protein formed after the expression of the mutant of GDH1 is shown in SEQ ID NO. 43, or a sequence having at least 70% homology therewith;
a nucleotide sequence of SuhB is as shown in SEQ ID NO. 23, or a sequence having at least 70% homology therewith; an amino acid sequence of a protein formed after the expression of SuhB is shown in SEQ ID NO. 44, or a sequence having at least 70% homology therewith.

9. Use of the recombinant yeast strain according to any one of claims 3 to 8 in the production of glucose, sucrose, glucosamine, inositol, analogs or derivatives of glucose, analogs or derivatives of sucrose, analogs or derivatives of glucosamine, and analogs or derivatives of inositol by means of biotransformation;
preferably, the analogs of glucose are isomers of glucose, and the derivatives of glucose comprise derivatives of alcohols, amines, oligosaccharides, and polysaccharides of glucose;
more preferably, the analogs of glucose comprise fructose and galactose, and the derivatives of glucose comprise sugar alcohol, ammonia sugar, disaccharide sucrose, and polysaccharide starch.

10. The use according to claim 9, wherein the recombinant yeast strain A or the recombinant yeast strain E is used to ferment a carbon source as a substrate in a culture medium to obtain glucose;
the recombinant yeast strain B or the recombinant yeast strain F is used to ferment a carbon source as a substrate in a culture medium to obtain glucosamine;
the recombinant yeast strain C or the recombinant yeast strain G is used to ferment a carbon source as a substrate in a culture medium to obtain sucrose;
the recombinant yeast strain D or the recombinant yeast strain H is used to ferment a carbon source as a substrate in a culture medium to obtain inositol;
preferably, the carbon source is a non-grain carbon source, comprising acetic acid, methanol, ethanol, propanol, and glycerol.
